(19) Europäisches Patentamt
European Patent Office
Office européen des brevets

(11) **EP 4 095 140 A1**

(12) **EUROPEAN PATENT APPLICATION**
published in accordance with Art. 153(4) EPC

(43) Date of publication:
**30.11.2022 Bulletin 2022/48**

(21) Application number: **21743962.9**

(22) Date of filing: **22.01.2021**

(51) International Patent Classification (IPC):
***C07D 487/04*** *(2006.01)* ***A61K 31/52*** *(2006.01)*
***A61P 1/16*** *(2006.01)*

(52) Cooperative Patent Classification (CPC):
**A61K 31/52; A61P 1/16; C07D 487/04**

(86) International application number:
**PCT/KR2021/000930**

(87) International publication number:
**WO 2021/150069 (29.07.2021 Gazette 2021/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.01.2020 KR 20200009486**

(71) Applicant: **Bioway. Inc.**
**Chuncheon-si, Gangwon-do 24232 (KR)**

(72) Inventors:
- **KIM, Jong Woo**
  **Uiwang-si Gyeonggi-do 16016 (KR)**
- **LEE, Chi Woo**
  **Gunpo-si Gyeonggi-do 15885 (KR)**
- **KWON, Hye Shin**
  **Uijeongbu-si Gyeonggi-do 11696 (KR)**
- **KWON, Sang Hoon**
  **Seoul 06668 (KR)**
- **JU, Ukil**
  **Seoul 05010 (KR)**
- **YOO, Yoonseon**
  **Sejong-si Sejong 30047 (KR)**
- **CHOI, Bo Ra**
  **Daejeon 34401 (KR)**

(74) Representative: **Dantz, Jan Henning et al**
**Loesenbeck - Specht - Dantz**
**Patent- und Rechtsanwälte**
**Am Zwinger 2**
**33602 Bielefeld (DE)**

(54) **NOVEL QUINAZOLINE COMPOUND AS THERAPEUTIC AGENT FOR METABOLIC DISORDERS**

(57) The present inventors have arrived at the present application by confirming that a novel quinazoline derivative exhibits therapeutic efficacy for metabolic disorders. The quinazoline derivative according to the present application has a similar structure to that of idelalisib, but exhibits very different properties and a different mechanism of action from idelalisib. In addition, this derivative molecule was shown to have excellent efficacy against metabolic disorders, in particular lipid metabolic disorders, through different mechanism of action. The derivative molecule also showed excellent efficacy against non-alcoholic steatohepatitis (NASH). No non-alcoholic steatohepatitis treatment drugs have been approved at the time of filing.

[FIG. 1]

FIG. 1

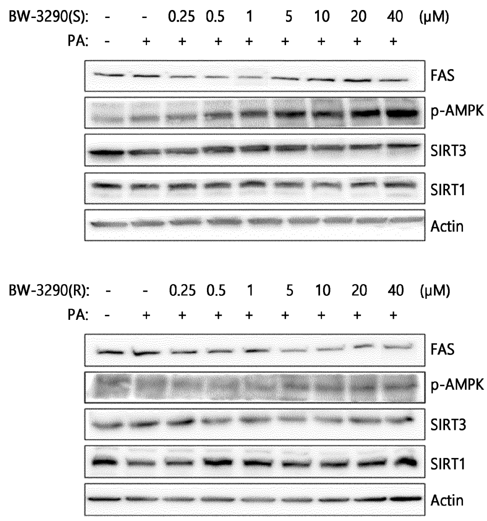


EP 4 095 140 A1

Processed by Luminess, 75001 PARIS (FR)

## Description

### Technical Field

The present invention relates to novel quinazolinone compounds that can be used for the treatment of metabolic disorders, in particular fatty liver.

### Background Art

Quinazolinone derivatives have been variously studied medically. Among them, idelalisib (Chemical Formula 2) is a substance that has been developed as a treatment for blood cancer and is used as a second-line drug for blood cancer.

[Chemical Formula 2]

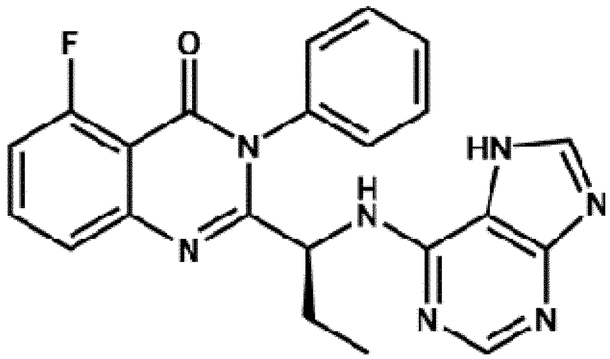

Metabolic disorder is a disease that commonly occurs in modern people and poses a great risk to the health of modern people. Various attempts are being made to treat metabolic disorders.

The inventors of the present invention have reached the present invention by confirming that the novel quinazolinone derivatives show an efficacy for treating metabolic disorders. Although the quinazolinone derivatives according to the present invention have a structure similar to that of idelalisib, but they exhibit properties extremely different from those of idelalisib and show a different mode of action. Additionally, the derivative molecules were shown to exhibit an excellent efficacy on metabolic disorders, particularly a lipid metabolic disorder, through the different mechanisms of action. The derivative molecules have also shown an excellent efficacy against non-alcoholic steatohepatitis (NASH). As of the filing date of the present application, there is no approved drug for the treatment of nonalcoholic steatohepatitis.

### Disclosure

### Technical Problem

The present invention provides novel quinazolinone derivatives.

The present invention provides pharmaceutical uses of the quinazolinone derivatives. Further, the present invention provides uses of the quinazolinone derivatives for treating metabolic disorders.

### Technical Solution

The present invention provides compounds represented by Chemical Formula 1 or pharmaceutically acceptable salts thereof:

[Chemical Formula 1]

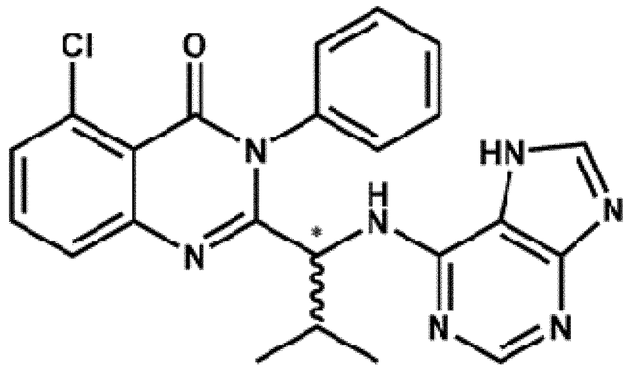

In particular, * is a stereocenter, and the compound is an S-form or R-form based on the stereocenter, or a racemate in which the S-form and the R-form are mixed.

Further, the present invention provides compounds represented by Chemical Formula 3 or Chemical Formula 4, or pharmaceutically acceptable salts thereof:

[Chemical Formula 3]

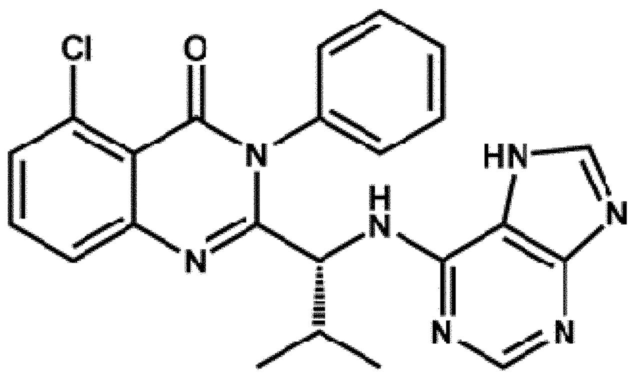

[Chemical Formula 4]

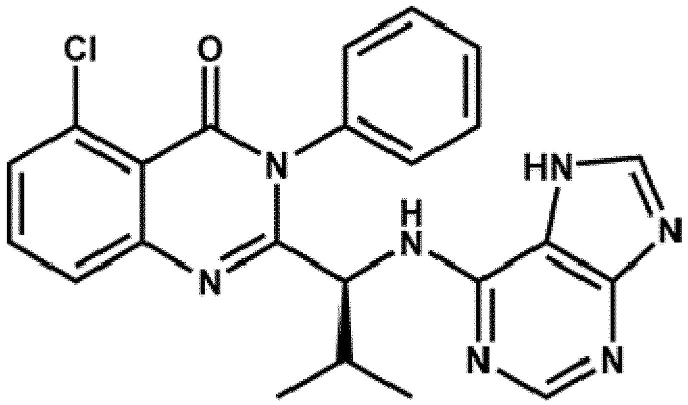

Alternatively, the present invention provides a pharmaceutically acceptable salt of the compound represented by Chemical Formula 1, characterized in that the pharmaceutically acceptable salt is a hydrochloride salt.

The present invention provides pharmaceutical compositions for treating metabolic disorders comprising the compound represented by Chemical Formula 1 or a pharmaceutically acceptable salt thereof:

[Chemical Formula 1]

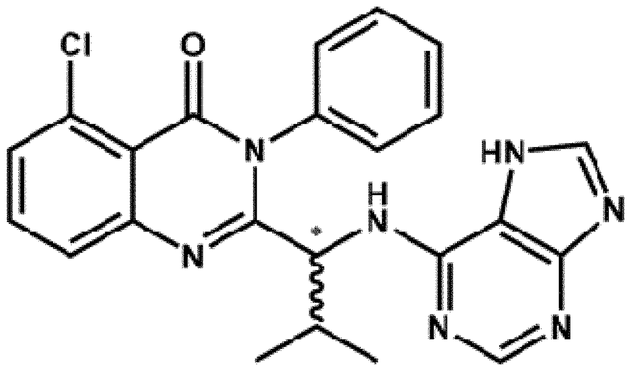

In particular, * is a stereocenter, and the compound is an S-form or R-form based on the stereocenter, or a racemate in which the S-form and the R-form are mixed.

Further, according to the present invention provides a pharmaceutical composition for treating a lipid metabolic disease, in which the lipid metabolic disease is selected from non-alcoholic fatty liver disease (NAFLD), non-alcoholic steatohepatitis (NASH), and liver fibrosis. Furthermore, the present invention provides a pharmaceutical composition comprising the compound represented by Chemical Formula 3 or Chemical Formula 4:

[Chemical Formula 3]

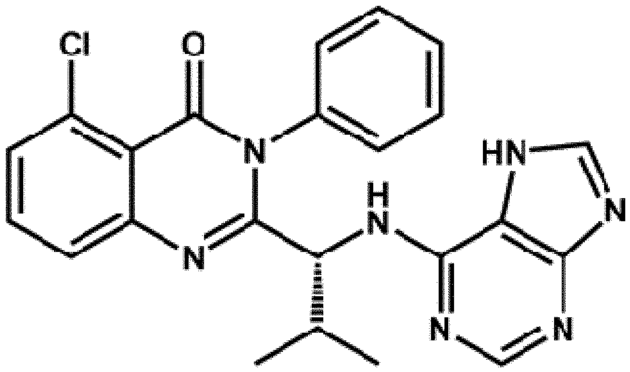

[Chemical Formula 4]

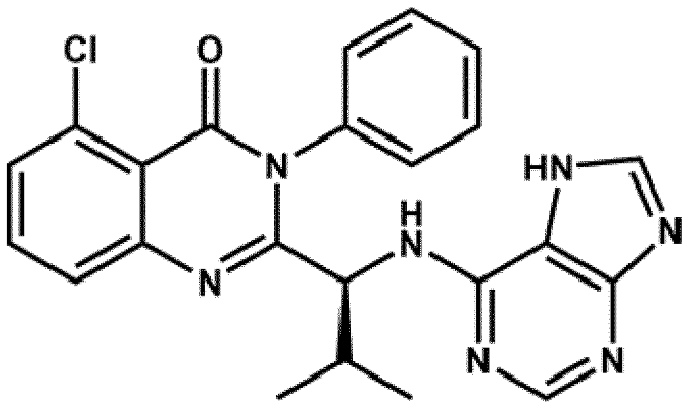

**Advantageous Effects**

As a result of administering the novel compound according to the present invention, a therapeutic efficacy for metabolic disorders was confirmed as described below. Further, the compound of the present invention was confirmed to have a therapeutic efficacy for metabolic disorders through phosphorylation of AMPK, activation of SIRT1, inhibition of inflammation, or a multiple pharmacological mechanism of two or more selected among these. Accordingly, it is expected that the compound of the present invention could provide more excellent treatment for metabolic disorders, and in particular, could provide a breakthrough treatment for nonalcoholic steatohepatitis for which no commercial drugs are available.

**Description of Drawings**

FIG. 1 shows experimental results regarding the expression levels of proteins in which the pharmacological mechanism of the compound according to the present invention was confirmed.
FIG. 2 shows experimental results regarding cytotoxicity of the compound according to the present invention.
FIG. 3 shows the effect of the compound according to the present invention on inhibition of fat accumulation compared to the existing developed material.
FIG. 4 shows experimental results of expression of the fat oxidation promoting protein by the compound of the present invention compared to the existing developed materials.
FIG. 5 shows experimental results in which the inhibition of fat accumulation in 3TL-L1 adipocytes was confirmed.
FIG. 6 shows the analysis of the results of FIG. 5.
FIG. 7 shows the results in which the inhibition of adipocyte formation in steatosis-induced hepatocytes.

FIG. 8 shows the analysis of the results of FIG. 7.

FIG. 9 shows the results confirming the expression of lipogenesis-related factors in steatosis-induced hepatocytes, in which (A) shows experimental results of expression of lipogenesis-related factors according to the concentration of the compound of the present invention, and (B) shows experimental results of quantitative analysis of mRNA in steatosis-induced Huh7 cells.

FIG. 10 shows the results confirming the expression of fatty acid beta-oxidation factors in steatosis-induced hepatocytes.

FIG. 11 shows the results confirming the expression of inflammatory factors in steatosis-induced hepatocytes.

FIG. 12 shows the results of quantitative analysis of mRNA of inflammatory factors in steatosis-induced hepatocytes.

FIG. 13 shows experimental results confirming the activity of the compound according to the present invention as a multiple mode regulator.

FIG. 14 shows the results of confirming the SIRT1 activity of the compound according to the present invention.

FIG. 15 shows the results of confirming the protein expression levels for p-AMPK and p-ACC, which show the activity of AMPK in the multiple mode activity of the compound of the present invention.

FIG. 16 shows the results of confirming PK by the compound of the present invention.

FIG. 17 shows the results of confirming the *in-vivo* activity of the compound according to the present invention.

FIG. 18 shows the results of confirming the *in-vivo* activity of the compound according to the present invention by a histopathological method.

FIG. 19 shows a histopathological diagnosis result showing the efficacy of *in-vivo* improvement of liver fibrosis by the compound according to the present invention.

FIG. 20 shows the results of exhibiting the effect of the compound according to the present invention on changes in insulin resistance.

FIG. 21 is a schematic diagram showing the pharmacological mechanism of the compound according to the present invention.

FIG. 22 shows the analysis of the results of FIG. 19.

## Modes of the Invention

### Definitions

Unless otherwise defined, all technical and scientific terms used in the present invention have the meaning commonly understood by those skilled in the art of the present invention. The following references provide those skilled in the art with general definitions of many of the terms used in the present invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As long as being used in the present invention, the following terms have the meanings conferred to them below, unless otherwise specified.

In some embodiments, chemical structures are disclosed along with corresponding chemical names. In case of a dispute, the meaning should be grasped by the chemical structure, taking precedence over the chemical name.

As used herein, the term "a pharmaceutically acceptable salt" refers to a salt that has the efficacy of a parent agent and is not biologically undesirable (e.g., is non-toxic or unharmful to its receptors). Suitable salts include acid salts which can be formed by mixing with a solution of a pharmaceutically acceptable acid, for example, an inorganic acid (*e.g.,* hydrochloric acid, phosphoric acid, sulfuric acid, *etc.)* and an organic acid (*e.g.,* methanesulfonic acid, *p*-toluenesulfonic acid, acetic acid, citric acid, maleic acid, succinic acid, oxalic acid, benzoic acid, tartaric acid, fumaric acid, manderic acid, glucuronic acid, trifluoroacetic acid, benzoic acid, *etc.).* When the drug carries an acidic moiety (*e.g.,* -COOH or a phenolic group), the pharmaceutically acceptable salt may include salts formed with suitable organic ligands such as alkali metal salts (*e.g*., sodium or potassium salts), alkaline earth metal salts (*e.g.*, calcium or magnesium salts), and quaternary ammonium salts.

<u>Treatment of metabolic disorders: SIRT1 activator</u>

In an aspect, the present invention provides a therapeutic agent for metabolic disorders comprising an SIRT1 activator. Additionally, in an aspect, the present invention provides a method of treating metabolic disorders comprising the step of administering an SIRT1 activator to a subject. Additionally, in an aspect, the present invention provides a molecule that function as an SIRT1 activator.

The molecule according to the present application can function as an activator of SIRT1 as shown in the Experimental Examples below. The SIRT1 activator can function as a therapeutic agent for metabolic disorders as described below.

Sirtuin 1 (hereinafter, SIRT1), which is NAD-dependent deacetylase, is known to control the deacetylation and activity of gene-regulatory proteins (*e.g.,* ChREBP, SREBP-1, PPARα,PGC-1α, NF-κB, *etc.)* as well as histones.

SIRT1 is known to be involved in fatty acid oxidation and hepatic lipid metabolism. Steatosis, which is an early stage of fatty liver disease, is characterized by the accumulation of excess triglycerides in the form of fat globules in the liver. One of the causes is that triglyceride-rich chylomicrons and free fatty acids enter the liver through transmembrane proteins. Another cause is that high levels of glucose and insulin stimulate the production of new lipids, and transcription factors (*e.g.,* ChREBP and SREBP1c) are activated, and they activate lipogenic enzymes (*e.g.,* FAS, ACC1, SCD1, and ELOVL6), and triglycerides and free fatty acids are synthesized in the liver. One of the pathways for removing these triglycerides and fatty acids is fatty acid beta-oxidation through the PPARα/PGC-1α signaling pathway in mitochondria and another is the release of triglycerides into the blood in the form of very low-density lipoprotein (VLDL). When SIRT1 is activated, it deacetylates ChREBP and SREBP1c, blocks the expression of lipogenesis-related genes by inhibiting new lipogenesis and may increase fatty acid beta-oxidation by deacetylation of PPARα/PGC-1α (Ding RB, Bao J, Deng CX. Int J Biol Sci. (2017) 13(7):852-867).

According to Purushotham A (Purushotham A, et al. Cell Metab. (2009) 9(4):327-38), liver cell-specific SIRT1 deletion impairs PPARα signaling and reduces fatty acid beta-oxidation. Additionally, when liver-specific SIRT1 knockout mice were fed with a high-fat diet, hepatic steatosis and hepatic inflammation were observed.

According to Choi SE (Choi SE, Kwon S, Seok S, et al. Mol Cell Biol. (2017) 37(15): e00006-17), when SIRT1 is phosphorylated by CK2 thereby reducing the activity of SIRT1, symptoms of fatty liver and fatty liver-related diseases (*e.g.,* a decrease of fat metabolism, an increase of adipogenesis, an increase of inflammatory responses *in vivo, etc.*) are exacerbated.

Considering the study results above, it is expected that the SIRT1 activating substance can be used for the prevention or treatment of fatty liver and fatty liver-related diseases.

SIRT1 activating substances are known to be effective in preventing or improving symptoms of obesity, NAFLD, diabetes, *etc.* Among the compounds known to activate SIRT1 is resveratrol. Resveratrol (3,4',5-trihydroxystilbene) is a polyphenol compound found in plants such as grapes and has a molecular formula of $C_{14}H_{12}O_3$. Studies on the effect of resveratrol on the prevention or treatment of heart disease, cancer, diabetes, and non-alcoholic fatty liver disease (NAFLD), *etc.* are being underway.

Treatment of metabolic disorders: AMPKactivator

In an aspect, the present invention provides a therapeutic agent for metabolic disorders comprising an AMPK activator. Additionally, in an aspect, the present invention provides a method of treating metabolic disorders comprising the step of administering an AMPK activator to a subject. Additionally, in an aspect, the present invention provides a molecule that function as an AMPK activator.

The molecule according to the present application can function as an activator of AMPK as shown in the Experimental Examples below. The AMPK activator can function as a therapeutic agent for metabolic disorders as described below.

AMP-activated protein kinase (AMPK) is known to function to maintain energy homeostasis in cells and promote glucose and fatty liver the uptake and oxidation when cellular energy is low. Specifically, AMPK is activated when the cellular energy decreases due to metabolic stress or exercise, inhibits ATP-consuming processes (fatty acid synthesis, cholesterol synthesis, *etc.),* and promotes ATP-producing processes (fatty acid oxidation, glycolysis, *etc.)* (Wing RR, Goldstein MG, Acton KJ, et al. Behavioral science research in diabetes: lifestyle changes related to obesity, eating behavior, and physical activity. Diabetes Care. 2001;24(1):117-123.). AMPK is known to be involved in the phosphorylation of acetyl-CoA carboxylase (ACC) *(i.e.,* a lipogenesis-related factor) and sterol regulatory element-binding protein 1c (SREBP1c) and inhibit lipogenesis (Jeon SM. Regulation and function of AMPK in physiology and diseases. Exp Mol Med. 2016;48(7):e245. Published 2016 Jul 15.). Additionally, AMPK activates hexokinase II *(i.e.,* a factor related to beta-oxidation of fatty acids), peroxisome proliferator-activated receptor alpha (hereinafter; PPARα), peroxisome proliferator-activated receptor delta (PPARδ), peroxisome proliferator-activated receptor delta coactivator-1 (PPARγ coactivator-1) (hereinafter; PGC-1), UCP-3, cytochrome C, and TFAM thereby promoting beta-oxidation. Focusing on such functions of AMPK, there was an idea to treat metabolic disorders through activation of AMPK (Winder WW, Hardie DG. AMP-activated protein kinase, a metabolic master switch: possible roles in type 2 diabetes. Am J Physiol. 1999;277(1):E1-E10.).

Treatment of metabolic disorders: anti-inflammatory agent

In an aspect, the present invention provides a therapeutic agent for metabolic disorders comprising an anti-inflammatory agent. Additionally, in an aspect, the present invention provides a method of treating metabolic disorders comprising the step of administering an anti-inflammatory agent to a subject. Additionally, in an aspect, the present invention provides a molecule that function as an anti-inflammatory agent. In this case, the anti-inflammatory agent may

inhibit inflammatory factors, for example, IκB kinase α(hereinafter; IKKα), interleukin 6 or 8 (hereinafter; IL6 or IL8), monocyte chemoattractant protein 1(hereinafter; MCP1), or Tumor necrosis factor α(hereinafter; TNFα).

The molecule according to the present application can function as an anti-inflammatory agent as shown in the Experimental Examples below. This is also due to the activation of AMPK and SIRT1. The anti-inflammatory agent can function as a therapeutic agent for metabolic disorders as described below.

Metabolic disorders and inflammation are known to be closely related. Inflammation is generally a beneficial phenomenon for returning tissue in an abnormal state to a normal state. However, some patients with a metabolic disease develop a condition of chronic inflammation in which the tissue is continuously inflamed as the tissue is not restored to a normal state. Prolonged inflammatory conditions, such as chronic inflammation, can be harmful to the human body. According to studies that have been conducted, the changes in the composition of immune cells in tissues due to inflammation can cause major symptoms of metabolic disorders including diabetes. For example, in diabetes, allegedly, the insulin secretion gland may be destroyed due to the cytokine secretion by inflammatory immune cells. Additionally, in the case of fatty liver, when the liver is damaged, Kupffer cells are activated to induce immune cells and thereby activate immune cells in the liver cells. Among them, hepatic stellate cells (hereafter; HSC cells) are known to promote the secretion and degradation of extracellular matrix proteins including fibrous collagen. The continuous activation of Kupffer cells and HSC cells is known to be one of the major causes of hepatic fibrosis (Bataller R, Brenner DA. Liver fibrosis [published correction appears in J Clin Invest. 2005 Apr;115(4): 1100]. J Clin Invest. 2005;115(2):209-218.). That is, the anti-inflammatory agent has the potential to improve the symptoms of fibrosis caused by metabolic disorders including liver fibrosis.

Treatment of metabolic disorders: multiple mode regulator

As used herein, the term "multiple mode regulator" refers to a drug or compound for which two or more modes of action expected to treat a target disease are confirmed. The molecule according to the present application can function as a multiple mode regulator for treating metabolic disorders. In particular, the modes of action of the multiple mode regulator may be selected from activators of SIRT1, activators of AMPK, and inhibition of inflammation as described above.

In an aspect, the present invention provides a therapeutic agent for metabolic disorders comprising a multiple mode regulator. Additionally, in an aspect, the present invention provides a method of treating metabolic disorders comprising the step of administering a multiple mode regulator to a subject. Additionally, in an aspect, the present invention provides a molecule that function as a multiple mode regulator.

In an embodiment, the multiple mode regulator can function as an SIRT1 activator and an AMPK activator. In an embodiment, the multiple mode regulator can function as an SIRT1 activator and an anti-inflammatory agent. Alternatively, in an embodiment, the multiple mode regulator can function as an SIRT1 activator, an AMPK activator, and an anti-inflammatory agent.

Structure of the compound according to the present application

In an aspect, the present invention provides compounds represented by Chemical Formula 1 or pharmaceutically acceptable salts thereof:

[Chemical Formula 1]

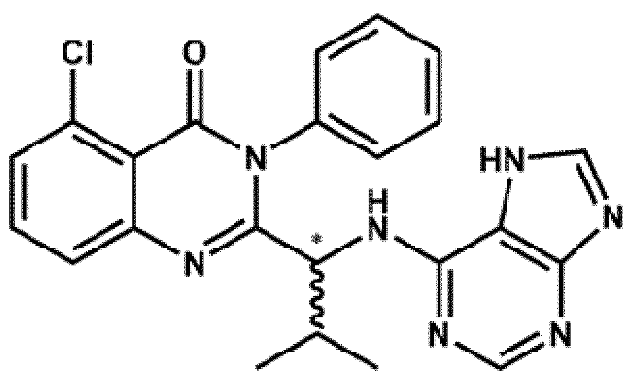

In particular, * is a stereocenter, and the compound is an S-form or R-form based on the stereocenter, or a racemate in which the S-form and the R-form are mixed.

This is to show that the wavy bond structure bound to a stereocenter may be a bond entering the plane or a bond coming out of the plane, depending on the isomer.

In an embodiment, the pharmaceutically acceptable salt may be an acid salt. In particular, the acid salt includes all forms that those skilled in the art can use, such as inorganic acids (*e.g.,* hydrochloric acid, phosphoric acid, sulfuric acid, *etc.)* and organic acids (*e.g.,* methanesulfonic acid, *p*-toluenesulfonic acid, acetic acid, citric acid, maleic acid, succinic acid, oxalic acid, benzoic acid, tartaric acid, fumaric acid, manderic acid, glucuronic acid, trifluoroacetic acid, benzoic acid, *etc.).* In a preferred embodiment, the pharmaceutically acceptable salt may be a hydrochloride salt.

*Differences from idelalisib*

The molecule according to the present invention shares a structure similar to that of the previously developed drug idelalisib. The structure of idelalisib is as shown in Formula 2 below.

[Chemical Formula 2]

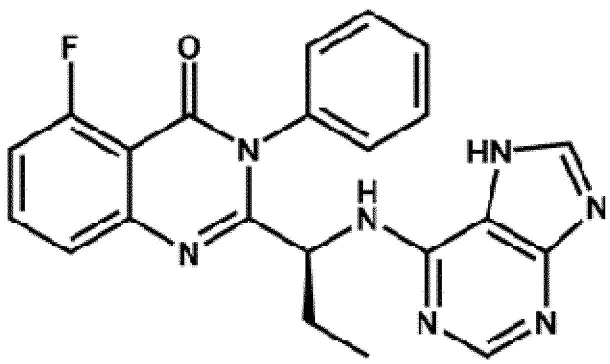

Idelalisib has been studied as a therapeutic agent for blood cancer and as an inhibitor of phosphoinositide 3-kinase inhibitor (PI3K).

However, the inventors of the present invention, while changing the molecular structure of idelalisib, identified one which it has a different mode of action than idelalisib and has efficacy against different diseases. Hereinafter, structural differences between idelalisib and the molecule of the present invention and what is meant by the differences will be described.

*Substitution of quinazoline structure*

The $R_1$ substituent is described here.

The inventors of the present invention confirmed that in Chemical Formula 1, the combination of halogen or $R_1$ having an equivalent size, and the "isopropyl" group introduced at the stereocentric carbon shows a different aspect of pharmaceutical efficacy compared to conventional idelalisib. That is, while idelalisib is limited to its efficacy on blood cancer, the quinazolinone derivatives of the present invention having the above-described characteristics showed pharmaceutical efficacy more specific to fatty liver-related diseases.

*Stereocenter*

The stereocenter of the compound is described here. The stereocenter exhibits different characteristics depending on the type of the substituent substituted therefor (hereinafter, a stereocenter substituent) and the shape of the isomer.

In the present invention, the stereocenter substituent is an isopropyl moiety. This corresponds to a new structure compared to the prior art such as idelalisib. Reviewing the prior art, the structure of the stereocenter substituent has a significant influence on the characteristics of the compound. For example, duvelisib is a quinazolinone derivative in which the stereocentric substituent is methyl. Despite such difference, duvelisib showed a characteristic in which the

selectivity for the isoform of PI3K is very lower than that of idelalisib. In another embodiment, another registered Korean Patent No. 10-1932146 of the same applicant discloses a compound wherein the stereocentric substituent is cyclopropyl or cyclobutyl. The compounds were shown to have improved treatment efficacy on blood cancer and selectivity for PI3K isoform compared to idelalisib.

The inventors of the present invention confirmed that when the stereocenter substituent is an isopropyl moiety, it has an excellent ability to regulate SIRT1, AMPK, and inflammatory factors contrary to what was previously known. Based on this, the inventors of the present invention confirmed that the novel molecule has an excellent effect on metabolic disorders.

In the present invention, the stereocenter may be of two isomeric types (S-type, R-type). The type of isomer has a significant influence on the characteristics of the present compound. Representatively, the type of isomer can affect the cytotoxicity of the compound and the aspect of the mode of action.

In the present invention, the stereocenter may be S-type or R-type. In an embodiment, the stereocenter may be an S-type. In another embodiment, the stereocenter may be an R-type. In another embodiment, the compound may be a racemate in which S-form and R-form are mixed around the stereogenic center.

Specific examples

In an aspect, the present invention provides a compound of Chemical Formula 3 or pharmaceutically acceptable salts thereof:

[Chemical Formula 3]

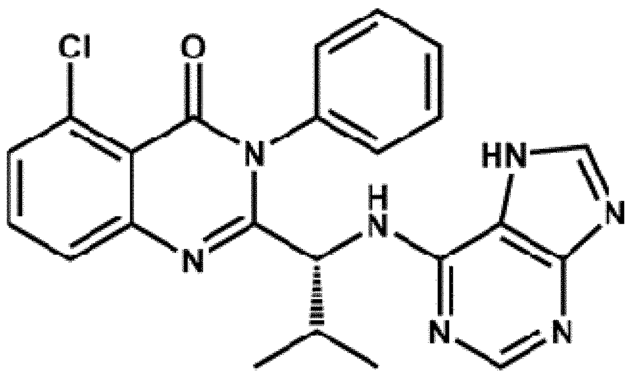

In an aspect, the present invention provides a compound of Chemical Formula 4 or pharmaceutically acceptable salts thereof:

[Chemical Formula 4]

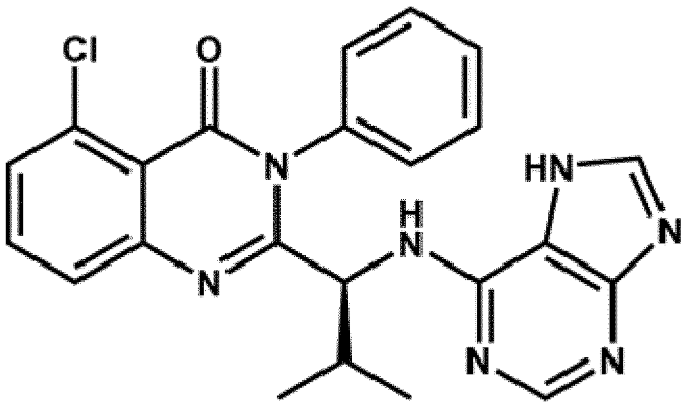

Therapeutic Use

In an aspect, therapeutic uses of one or more compounds selected from Chemical Formulas 1, 3, and 4 for metabolic disorders is provided by the present invention.

In an aspect, a pharmaceutical composition containing one or more compounds selected from Formulas 1, 3, and 4 is provided by the present invention. The pharmaceutical composition may contain a pharmaceutically acceptable excipient. In the pharmaceutical composition according to the present invention, the compound according to the present invention, or a pharmaceutically acceptable salt thereof, may be administered in various formulations according to oral and/or parenteral methods during clinical administration. In particular, when formulating a compound or a pharmaceutically acceptable salt thereof, the compound or a pharmaceutically acceptable salt thereof may be formulated using at least one of a diluent or excipient such as a filler, an extender, a binder, a wetting agent, a disintegrant, and a surfactant.

In an embodiment, the formulation for oral administration containing the compound according to the present invention or a pharmaceutically acceptable salt thereof as an active ingredient may include tablets, pills, hard/soft capsules, liquid preparations, suspensions, emulsifiers, syrups, granules, elixirs, troches, *etc.* The formulation for oral administration may include, in addition to the active ingredient, at least one among the diluents (*e.g.,* lactose, dextrose, sucrose, mannitol, sorbitol, cellulose and/or glycic), and lubricants (*e.g.,* silica, talc, stearic acid and a magnesium or calcium salt thereof and/or polyethylene glycol). For example, the tablet may contain at least one among the binders (*e.g.,* magnesium aluminum silicate, starch paste, gelatin, methylcellulose, sodium carboxymethylcellulose and/or polyvinylpyrrolidine, *etc.).* Additionally, the tablet may contain, depending on the case, at least one of starch, agar, a disintegrant (*e.g.,* alginic acid or a sodium salt thereof) or an azeotrope and/or an absorbent, a coloring agent, a flavoring agent, and a sweetening agent.

In an embodiment, the pharmaceutical composition containing the compound according to the present invention or a pharmaceutically acceptable salt thereof as an active ingredient may be used as a method for parenteral administration. The parenteral administration method may be any one of the injection methods among subcutaneous injection, intravenous injection, intramuscular injection, and intrathoracic injection.

In an embodiment, the formulation for parenteral administration containing the compound according to the present invention or a pharmaceutically acceptable salt thereof as an active ingredient may be prepared as a solution or suspension by mixing in water with a stabilizing agent or buffer, and it may be prepared in ampoules or in a vial unit dosage form. Additionally, the formulation for parenteral administration containing the compound according to the present invention or a pharmaceutically acceptable salt thereof as an active ingredient may contain adjuvants (*e.g.,* preservatives, stabilizing agents, wetting agents or emulsifying agents, salts and/or buffers for regulating osmotic pressure) and other therapeutically useful substances, and may be formulated according to mixing, granulation or coating in a conventional manner. Additionally, the pharmaceutical composition according to the present invention may be prepared in a formulation for sustained release of a compound or a pharmaceutically acceptable salt thereof through a polymer excipient, *etc.*

Additionally, the pharmaceutical composition of the present invention may contain one or more active ingredients that can exhibit the same or similar function, in addition to the compound according to the present invention or a pharmaceutically acceptable salt thereof.

Additionally, the preferred dose of the pharmaceutical composition of the present invention may be appropriately selected according to the patient's condition and weight, the severity of symptoms, drug form, administration route, and period. For optimal efficacy of the pharmaceutical composition of the present invention, it may be desirable that the

active ingredient be administered at 0.2 mg/kg to 200 mg/kg per day. Additionally, the pharmaceutical composition of the present invention may be administered once a day, and may be administered in several divided doses, but is not limited thereto.

In an aspect, the present invention provides a method of treating metabolic disorders which includes administering to a subject one or more compounds selected from Formulas 1, 3, and 4. In this case, the compound may be administered in the form of an appropriate composition in an effective dose via an appropriate route. Such routes, compositions, and doses may be determined by a method known in the art.

In an aspect, the present invention provides a health functional food composition containing one or more compounds selected from Formulas 1, 3, and 4.

Target Disease: Metabolic disorders

The target diseases for the above-described therapeutic uses are described here. The compound of the present invention, or a pharmaceutically acceptable salt thereof, and a therapeutic use thereof are directed to metabolic disorders; in particular, are directed to lipid metabolism among metabolic disorders. The compound according to the present invention has the effects of promoting lipid degradation and inhibiting lipogenesis, and thus can improve abnormal lipid metabolism.

In an aspect, the present invention provides a pharmaceutical composition for treating metabolic disorders. In an aspect, the present invention provides a health functional food composition for treating metabolic disorders. In an aspect, the present invention provides methods for treating metabolic disorders.

Exemplary lipid metabolic disorders include steatosis, liver disease, obesity, diabetes, hypertension, hypercholesterolemia, insulin resistance, *etc.* Exemplary liver diseases include fatty liver, such as alcoholic liver disease and non-alcoholic fatty liver disease (NAFLD). Non-alcoholic fatty liver includes simple fatty liver and non-alcoholic steatohepatitis (NASH), and complications due to fatty liver (*e.g.,* liver fibrosis, cirrhosis, liver cancer, esophageal varices, *etc.).*

## Experimental Examples

### Experimental Example 1. Synthesis of compounds according to the invention

### Experimental Example 1.1. Method for preparing 2-(1-((7H-purin-6-yl)amino)-2-methylpropyl)-5-chloro-3-phenylquinazolin-4(3H)-one

[Reaction Scheme 1]

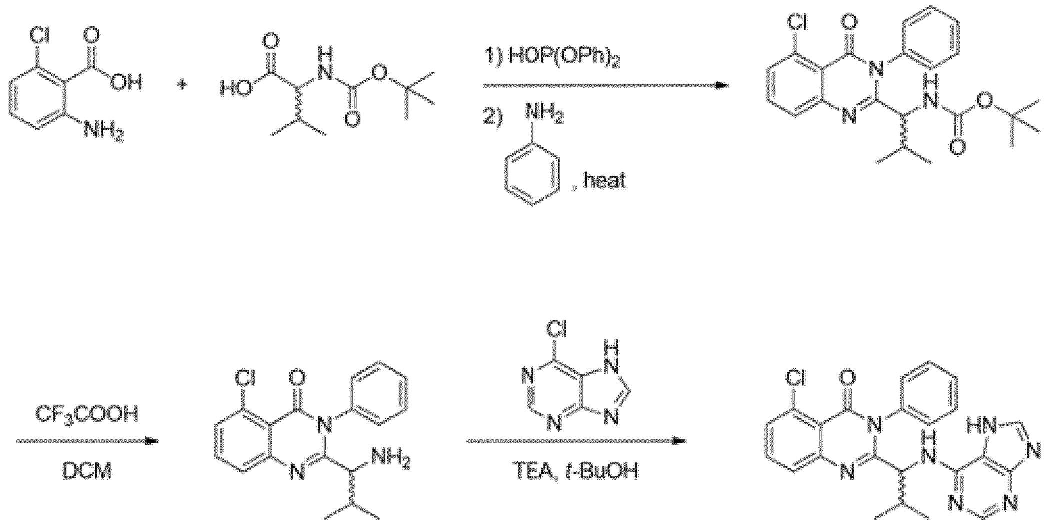


Step 1: Preparation of *tert*-butyl(1-(5-chloro-4-oxo-3-phenyl-3,4-dihydroquinazolin-2-yl)-2-methylpropyl)carbamate

While stirring a solution in which 2-amino-6-chlorobenzoic acid (2.82 g, 1.0 eq) and 2-((*tert*-butoxycarbonylamino)-3-methylbutanoic acid (5 g) were mixed in a pyridine solvent (30 mL) at room temperature, diphenyl phosphite (19 mL) was added thereto. The resulting mixture was stirred at 45-50°C for 3 hours, and aniline (18 mL) was added and reacted at 45-50°C for 1.5 hours. Then, the reaction mixture was cooled to room temperature and extracted with ethyl acetate and water. The organic layer was dehydrated with anhydrous magnesium sulfate ($MgSO_4$) and concentrated under reduced pressure. The residue was purified by column chromatography, and the obtained solid was dried to *tert*-butyl(1-(5-chloro-4-oxo-3-phenyl-3,4-dihydroquinazolin-2-yl)-2-methylpropyl)carbamate was obtained in 81% yield (5.72 g).

Step 2: Preparation of 2-(1-amino-2-methylpropyl-5-chloro-3-phenylquinazolin-4(3H)-one

Trifluoroacetic acid (39 mL) was added to a dichloromethane (157 mL) solution in which (S)-*tert*-butyl(1-(5-chloro-4-oxo-3-phenyl-3,4-dihydroquinazolin-2-yl)-2-methylpropyl)carbamate (5.72 g) was dissolved. The reaction solution was stirred at room temperature for about 0.5 to 1 hour, and then the pH of the reaction solution was adjusted to about pH 10 using aqueous ammonia. The reaction mixture was extracted with dichloromethane and water, and the organic layer was dehydrated and filtered using anhydrous magnesium sulfate ($MgSO_4$). After removing the anhydrous magnesium sulfate ($MgSO_4$), the filtrate was concentrated under reduced pressure. After purifying the residue by column chromatography, the obtained solid was dried to give 2-(1-amino-2-methylpropyl-5-chloro-3-phenylquinazolin-4(3H)-one in 88% yield (3.88 g).

Step 3: Preparation of 2-(1-((7H-purin-6-yl)amino)-2-methylpropyl)-5-chloro-3-phenylquinazolin-4(3H)-one

After adding 2-(1-amino-2-methylpropyl-5-chloro-3-phenylquinazolin-4(3H)-one (3.88g) obtained in Step 2 to *tert*-butanol (80 mL), triethylamine (3.3 mL) and 6-chloro-9H-purine (3.66 g) were added thereto, and stirred while refluxing the reaction solution for 36 hours. The reaction mixture was cooled and extracted with dichloromethane and water. The organic layer was dehydrated with anhydrous magnesium sulfate ($MgSO_4$) and concentrated under reduced pressure. The residue was purified by column chromatography, and the obtained solid was dried to 2-(1-((7H-purin-6-yl)amino)-2-methylpropyl)-5-chloro-3-phenylquinazolin-4(3H)-one was obtained in 68% yield (3.46 g).

$^1$HNMR(400 MHz, $CDCl_3$) $\delta$ 14.03 (brs, 1H), 8.31 (s, 1H), 7.99(s, 1H), 7.63-7.53(m, 5 H), 7.45-7.43 (dd, J = 7.6Hz, 1.6Hz, 1 H), 7.38-7.36 (d, J = 7.6Hz, 1 H), 7.34-7.2 (m, 1 H), 6.66-6.63 (d,J=9.2Hz, 1 H), 5.30-5.28 (m, 1 H), 2.33-2.25 (m, 1H), 0.98-0.9 (d, J = 6.8Hz, 3 H), 0.87-0.85 (d, J=6.8Hz,3H)

ESI-MS m/z 446.32 $[M+H]^+$

**Experimental Example 1.2. Method for preparing (S)-2-(1-((7H-purin-6-yl)amino)-2-methylpropyl)-5-chloro-3-phenylquinazolin-4(3H)-one**

[Reaction Scheme 2]

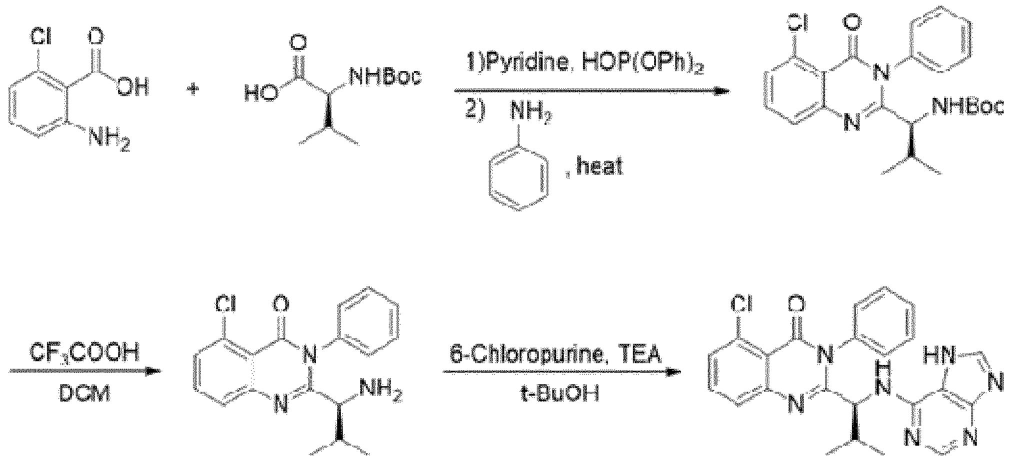

Step 1: Preparation of (S)-*tert*-butyl(1-(5-chloro-4-oxo-3-phenyl-3,4-dihydroquinazolin-2-yl)-2-methylpropyl)carbamate

While stirring a solution in which 2-amino-6-chlorobenzoic acid (0.2 g) and (S)-2-((*tert*-butoxycarbonylamino)-3-methylbutanoic acid (0.33 g) were mixed in a pyridine solvent (1.2 mL) at room temperature, diphenyl phosphite (0.34 mL) was added thereto. The resulting mixture was stirred at 45-50°C for 4 hours, and aniline (0.11 mL) was added and reacted at 45-50°C for 12 hours. Then, the reaction mixture was cooled to room temperature and extracted with ethyl acetate and water. The organic layer was dehydrated with anhydrous magnesium sulfate ($MgSO_4$) and concentrated under reduced pressure. The residue was purified by column chromatography, and the obtained solid was dried to (S)-*tert*-butyl(1-(5-chloro-4-oxo-3-phenyl-3,4-dihydroquinazolin-2-yl)-2-methylpropyl)carbamate was obtained in 37% yield (0.18 g).

1H NMR (500 MHz, CDCl3):δ7.62-7.61(m,2H), 7.59-7.51(m,3H), 7.48-7.46(m,1H), 7.32-7.28(m,2H), 5.38-5.36(m,1H), 4.39-4.36(m,1H), 2.03-1.97(m,1H), 1.42(s,9H), 0.84-0.83(d,J=6.5Hz, 3H), 0.72-0.71 (d,J=7Hz, 3H).

Step 2: Preparation of (S)-2-(1-amino-2-methylpropyl-5-chloro-3-phenylquinazolin-4(3H)-one

Trifluoroacetic acid (3.75 mL) was added to a dichloromethane (15 mL) solution in which (S)-*tert*-butyl(1-(5-chloro-4-oxo-3-phenyl-3,4-dihydroquinazolin-2-yl)-2-methylpropyl)carbamate (0.54 g) was dissolved. The reaction solution was stirred at room temperature for about 0.5 to 1 hour, and then the pH of the reaction solution was adjusted to about pH 10 using aqueous ammonia. The reaction mixture was extracted with dichloromethane and water, and the organic layer was dehydrated and filtered using anhydrous magnesium sulfate ($MgSO_4$). After removing the anhydrous magnesium sulfate ($MgSO_4$), the filtrate was concentrated under reduced pressure. After purifying the residue by column chromatography, the obtained solid was dried to give (S)-2-(1-amino-2-methylpropyl-5-chloro-3-phenylquinazolin-4(3H)-one in 36% yield (0.15 g).

1H NMR (400 MHz, CDCl3):δ7.64-7.48(m,5H),7.46-7.44(dd,J=6.4Hz, 2.4Hz, 1H), 7.29-7.25 (m, 2H), 3.25-3.23 (d, J=6.4Hz, 1H), 2.06-1.98 (m, 1H), 1.71 (brs, 2H), 0.88-0.86 (d, J=6.8Hz, 3H), 0.75-0.73 (d,J=6.8Hz, 3H).

Step 3: Preparation of (S)-2-(1-((7H-purin-6-yl)amino)-2-methylpropyl)-5-chloro-3-phenylquinazolin-4(3H)-one

After adding (S)-2-(1-amino-2-methylpropyl-5-chloro-3-phenylquinazolin-4(3H)-one (0.15 g) obtained in Step 2 to *tert*-butanol (3 mL), triethylamine (0.13 mL) and 6-chloro-9H-purine (0.14 g) were added thereto, and stirred while refluxing the reaction solution for 24 hours. The reaction mixture was cooled and extracted with dichloromethane and water. The organic layer was dehydrated with anhydrous magnesium sulfate ($MgSO_4$) and concentrated under reduced pressure. The residue was purified by column chromatography, and the obtained solid was dried to (S)-2-(1-((7H-purin-6-yl)amino)-2-methylpropyl)-5-chloro-3-phenylquinazolin-4(3H)-one was obtained in 73% yield (0.15 g).

1H NMR (400 MHz, CDCl3):δ13.87(brs,1H), 8.31(s,1H), 7.99(brs,1H), 7.62-7.53(m,5H), 7.46-7.44(dd,J=7.6Hz, 0.8Hz, 1H), 7.38-7.36 (m, 1H), 7.33-7.32 (m, 1H), 6.63-6.61 (d, J=9.2Hz, 1H), 5.29 (m, 1H), 2.32-3.24 (m, 1H), 0.98-0.97 (d,J=6.8Hz, 3H), 0.86-0.85 (d, J=6.8Hz, 3H).

ESI-MS m/z 446.24 [M+H]+

**Experimental Example 1.3. Method for preparing (R)-2-(1-((7H-purin-6-yl)amino)-2-methylpropyl)-5-chloro-3-phenylquinazolin-4(3H)-one**

[Reaction Scheme 3]

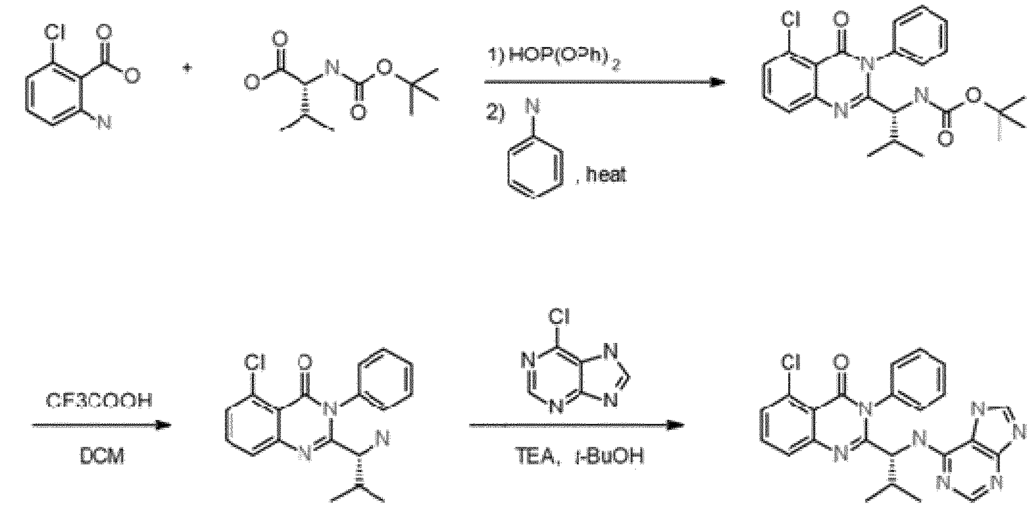


Step 1: Preparation of (R)-*tert*-butyl(1-(5-chloro-4-oxo-3-phenyl-3,4-dihydroquinazolin-2-yl)-2-methylpropyl)carbamate

While stirring a solution in which 2-amino-6-chlorobenzoic acid (0.2 g, 1.0 eq) and (R)-2-((*tert*-butoxycarbonylamino)-3-methylbutanoic acid (0.98 g) were mixed in a pyridine solvent (20 mL) at room temperature, diphenyl phosphite (3.7 mL) was added thereto. The resulting mixture was stirred at 45-50°C for 5 hours, and aniline (0.35 mL) was added and reacted at 45-50°C for 2 hours. Then, the reaction mixture was cooled to room temperature and extracted with ethyl acetate and water. The organic layer was dehydrated with anhydrous magnesium sulfate ($MgSO_4$) and concentrated under reduced pressure. The residue was purified by column chromatography, and the obtained solid was dried to (R)-*tert*-butyl(1-(5-chloro-4-oxo-3-phenyl-3,4-dihydroquinazolin-2-yl)-2-methylpropyl)carbamate was obtained in 74% yield (1.03 g).

$^1$H NMR (400 MHz, $CDCl_3$) δ 7.61-7.49 (m, 5 H), 7.47-7.45 (m, 1H), 7.32-7.28 (m, 2H), 5.38-5.36 (d, J = 9.2 Hz, 1H), 4.39-4.36 (m, 1 H), 2.04-1.96 (m, 1 H), 1.42 (s, 9H), 0.84-0.83 (d, J = 6.8Hz, 3H), 0.72-0.71 (d, J = 6.8Hz, 3H)

Step 2: Preparation of (R)-2-(1-amino-2-methylpropyl-5-chloro-3-phenylquinazolin-4(3H)-one

Trifluoroacetic acid (7 mL) was added to a dichloromethane (28 mL) solution in which (S)-*tert*-butyl(1-(5-chloro-4-oxo-3-phenyl-3,4-dihydroquinazolin-2-yl)-2-methylpropyl)carbamate (1.03 g) was dissolved. The reaction solution was stirred at room temperature for about 0.5 to 1 hour, and then the pH of the reaction solution was adjusted to about pH 10 using aqueous ammonia. The reaction mixture was extracted with dichloromethane and water, and the organic layer was dehydrated and filtered using anhydrous magnesium sulfate ($MgSO_4$). After removing the anhydrous magnesium sulfate ($MgSO_4$), the filtrate was concentrated under reduced pressure. After purifying the residue by column chromatography, the obtained solid was dried to give (R)-2-(1-amino-2-methylpropyl-5-chloro-3-phenylquinazolin-4(3H)-one in 93% yield (0.74 g).

$^1$HNMR(400 MHz, $CDCl_3$) δ 7.63-7.48 (m, 5 H), 7.47-7.45 (dd, J = 6.4 Hz, 2.4Hz, 1 H), 7.28-7.26 (m, 2 H), 3.25-3.23 (d, J = 6 Hz, 1 H), 2.06-1.98 (m, 1 H), 1.66 (brs, 2H), 0.88-0.87 (d, J = 6.8Hz,3H),0.75-0.73(d,J=6.8Hz,3H)

Step 3: Preparation of (R)-2-(1-((7H-purin-6-yl)amino)-2-methylpropyl)-5-chloro-3-phenylquinazolin-4(3H)-one

After adding (S)-2-(1-amino-2-methylpropyl-5-chloro-3-phenylquinazolin-4(3H)-one (0.74 g) obtained in Step 2 to *tert*-butanol (15 mL), triethylamine (0.63 mL) and 6-chloro-9H-purine (0.7 g) were added thereto, and stirred while refluxing the reaction solution for 24 hours. The reaction mixture was cooled and extracted with dichloromethane and water. The organic layer was dehydrated with anhydrous magnesium sulfate ($MgSO_4$) and concentrated under reduced pressure. The residue was purified by column chromatography, and the obtained solid was dried to (S)-2-(1-((7H-purin-6-yl)amino)-2-methylpropyl)-5-chloro-3-phenylquinazolin-4(3H)-one was obtained in 53% yield (0.534 g).

$^1$HNMR (400 MHz, $CDCl_3$) δ13.80 (brs, 1 H), 8.30 (s, 1 H), 7.99 (s, 1H), 7.64-7.54(m, 5 H), 7.46-7.44 (dd, J= 1.2Hz, 7.6Hz, 1H), 7.38-7.36 (d, J = 8Hz, 1H), 7.32-7.32 (m, 1 H), 6.63-6.60 (d, J = 8.8Hz, 1H), 5.29(m, 1H), 2.32-2.24 (m, 1H), 0.98-0.97 (d, J = 6.8 Hz, 3 H), 0.86-0.85 (d, J = 6.8 Hz, 3 H)

ESI-MS m/z 446.24 [M+H]+

**Experimental Example 1.4. Method for preparing (R)-2-(1-((7H-purin-6-yl)amino)-2-methylpropyl)-5-chloro-3-phenylquinazolin-4(3H)-one hydrochloride**

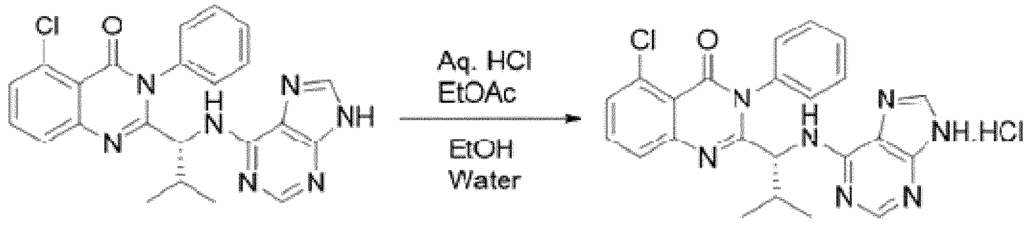


BW-3290 BW-3290 HCl

Ethyl acetate (2 mL) was added to (R)-2-(1-((7H-purin-6-yl)amino)-2-methylpropyl)-5-chloro-3-phenylquinazolin-4(3H)-one (0.5g) and cooled to 5-10°C. After adjusting the pH to about pH 1-2 using a 4 N aqueous hydrochloric acid solution, the reaction temperature was raised to 25-35°C and stirred for 2-3 hours. The resulting solid was filtered, washed with ethyl acetate to obtain (R)-2-(1-((7H-purin-6-yl)amino)-2-methylpropyl)-5-chloro-3-phenylquinazoline-4(3H)-one hydrochloride in 87% yield (0.47 g).

1HNMR (400 MHz, DMSO-d6) 88.81 - 8.45 (m, 3H), 7.78 (t, J = 8.0 Hz, 1H), 7.68 (d, J = 8.0 Hz, 1H), 7.63 - 7.46 (m, 6H), 5.00 (br. s., 1H), 2.44 - 2.35 (m, 1H), 0.99 -0.98 (d, J = 6.0 Hz, 3H), 0.84-0.83 (d, J = 6.8Hz, 3H)

Experimental Example 2. Confirmation of pharmacological mode of action (MOA) of the compounds according to the present invention

**Experimental Example 2.1. Experimental Method**

An experiment to confirm the expression levels of proteins in steatosis-induced liver cells was conducted as follows.

Huh7 Cell Preparation

To determine the protein expression level for MOA confirmation in steatosis-induced liver cells BW-3290 of the present invention, Huh7 cells were cultured in an incubator set at 37°C, 95% humidity, and 5% $CO_2$.

Additionally, Huh7 cells were proliferated to a confluency of 80-90% on a plate using a growth medium(Dulbecco ' s modified Eagle ' s medium (DMEM) supplemented with 10% fetal bovine serum (FBS), 100 U/ml of penicillin, and 100 µg/ml of streptomycin), and then used for the experiment.

Confirmation of expression level of proteins in steatosis-induced liver cells

To induce steatosis in Huh-7 cells in each well except for control cells, the Huh-7 cells were treated with PA bound to 2% BSA at a concentration of 250 µM except for the control, and the control cells were pretreated with 2% BSA without FA.

The cells in each well were treated with the compounds of the present invention, R- or S- form of BW-3290, 0.25 µM, 0.5 µM, 1 µM, 5 µM, 10 µM, 20 µM, and 40 µM and then cultured for 24 hours. The control cells and PA treated cells were treated with DMSO. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), 2 µg/mL aprotinin, 1 µM pepstatin A, 10 µM leupeptin, 1 mM sodium fluoride (NaF), 0.2 mM sodium orthovanadate ($Na_2VO_4$), and 1 µM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected, 5X sodium dodecyl sulfate (SDS) sample buffer containing 50 mM Tris-Cl (pH 6.8), 2% SDS, 0.1% bromopheol blue, and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membrane of fatty acid synthase (FAS), phosphor-AMP-activated protein kinase (p-AMPK), SIRT1, and SIRT3 was washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

**Experimental Example 2.2. Experimental Results**

Experimental results confirming the protein expression level by BW-3290 in steatosis-induced Huh7 cells are shown in FIG. 1.

FIG. 1 shows the results of confirming the protein expression levels relating to the pharmacological mechanism in steatosis-induced Huh7 cells according to the R-, S-form of BW-3290. That is, it shows the results of confirming the protein expression levels for FAS, which is involved in fat biosynthesis for Huh7 cells, and p-AMPK, SIRT1, and SIRT3, which are the core of MOA.

As can be seen in FIG. 1, it was confirmed that the level of FAS *(i.e.,* a fat synthesis protein) decreased in a concentration-dependent manner as the concentration increased in R-form of BW-3290, which is the compound of the present invention.

Additionally, it was confirmed that the proteins involved in the pharmacological effect of BW-3290 *(i.e.,* p-AMPK, SIRT1, and SIRT3) were increased in both S-form and R-form.

Therefore, it was confirmed that BW-3290, which is the compound of the present invention, is a novel compound that can be used for the treatment of metabolic disorders and fatty liver according to the pharmacological mechanism of AMPK and SIRT1.

**Experimental Example 3. Experimental Results of Cytotoxicity**

**Experimental Example 3.1. Experimental Method**

An experiment to determine whether the compounds exhibit cytotoxicity in normal cells was performed as follows.

<u>Preparation of MRC5 cells</u>

For confirmation of cytotoxicity in normal cells, MRC *(i.e.,* normal lung cells) were cultured in an incubator set at 37°C with 95% humidity and 5% $CO_2$.

Additionally, MRC5 cells were proliferated to a confluency of 70-80% on a plate using a growth medium(Minimum Essential Medium (MEM) supplemented with 10% fetal bovine serum (FBS), 100 U/ml of penicillin, and 100 μg/ml of streptomycin), and then used for the experiment.

<u>Confirmation of Cytotoxicity in MRC5 Cells</u>

In order to confirm the cytotoxicity of the compound according to an embodiment of the present invention, CCK8 analysis was performed for the prepared MRC5 cells.

First, after culturing MRC5 cells to 70-80% confluency in a 24-well plate, the test material was diluted in a

culture medium except for the control group and treated with 0.5 μM, 2.5 μM, 10 μM, 20 μM, 40 μM, 80 μM, and 100 μM.

After 24 hours of treatment of the test material, CCK8 solution was dispensed 10 μL per well and incubated for 2 hours in a state where the light was blocked.

After 2 hours, 200 μL of the culture solution from each well was transferred to a 96 well plate and absorbance was measured at 450 nm.

**Experimental Example 3.2. Experimental Results**

Experimental results confirming the cytotoxicity in normal cells are shown in FIG. 2. FIG. 2 is a drawing showing experimental results confirming the cytotoxicity in MRC5 cells through CCK8 analysis. However, for the convenience of the experiment, the Experimental Results are presented only for the cells shown in FIG. 2, and the Experimental results confirming cytotoxicity in normal cells are not limited to the cells shown in FIG. 2, and the cytotoxicity in normal cells can also be confirmed for cells corresponding to other normal cells. Experimental Results will be described in more detail in the content to be described later.

As shown in FIG. 2, whether normal cells have cytotoxicity was confirmed in normal cells using MRC5 cells according to the form of the compound of the present invention. As a result, it was confirmed that compared with the control group, the R-form compound did not show toxicity in normal cells, and the S-form and RS-form showed some toxicity. As a result of the experiment, the R-form compound of the compound according to the present invention was determined not to be toxic when used as a pharmaceutical composition.

**Experimental Example 4. Verification of inhibition of fat accumulation by the compound of the present invention compared to existing developed materials**

**Experimental Example 4.1. Experimental Method**

Huh7 Cell Preparation

In order to confirm the inhibition of fat accumulation in steatosis-induced liver cells, Huh7 cells were cultured in an incubator set at 37°C, 95% humidity, and 5% $CO_2$.

Additionally, Huh7 cells were proliferated to a confluency of 80-90% on a plate using a growth medium(Dulbecco ' s modified Eagle ' s medium (DMEM) supplemented with 10% fetal bovine serum (FBS), 100 U/ml of penicillin, and 100 μg/ml of streptomycin), and then used for the experiment.

Confirmation of fat globule reducing effect in steatosis-induced Huh7 cells through Oil-Red-O staining

In order to confirm the ability of BW-3290 to inhibit fat globule accumulation in steatosis-induced liver cells compared to the existing material under development, the liver cells were treated with palmitic acid (PA) bound to 2% BSA at a concentration of 250 μM except for the control group to induce steatosis, and then fat globules were stained through Oil-Red-O staining.

First, liver cells were cultured to grow 80-90% confluency in a 12 well plate, and the test material, BW-3290, were diluted in the culture medium that induced steatosis and the liver cells, except for the control group, were treated with 0.5 μM, 2.5 μM, 5 μM, and 10 μM of BW-3290.

Additionally, after removing the cell supernatant from the wells for Oil-Red-O staining, the washing process was performed twice with PBS.

Additionally, after washing, the resultant was fixed with 4% paraformaldehyde for 30 minutes.

Additionally, then, 60% 1,2-propanediol dehydration solution was added thereto and the mixture was incubated for 5 minutes.

Additionally, 5 minutes thereafter, the solution was removed and mix with Oil-Red-O coloring reagent (the ORO stock solution and distilled water were mixed at a ratio of 6 mL to 4 mL, and the mixture was filtered with a 0.45 μm filter to prepare the ORO solution) was added thereto to stain fat globules for 30 minutes.

Additionally, after staining, the remaining color reagent was removed by washing with PBS, and the formation of fat globules was observed under a microscope.

**Experimental Example 4.2. Experimental Results**

The experimental results confirming the effect of reducing fat accumulation in steatosis-induced Huh7 cells are shown in FIG. 3. In particular, FIG. 3 shows the inhibition of fat globule formation by the compound of the present invention confirmed through Oil-Red-O staining in comparison with the existing materials. However, for the convenience

of the experiment, the experimental results are presented only for the cells shown in FIG. 3, and the experimental results confirming the effect of reducing fat accumulation in adipocytes are not limited to the cells shown in FIG. 3, and the effect of reducing fat accumulation in adipocytes can also be confirmed for cells corresponding to cells inducing liver disease.

As shown in FIG. 3, it can be confirmed that the compound of the present invention has a lower level of fat globule staining compared to PA in all test groups. Considering that the experiment was performed by increasing the concentration of the compound to 0.5 μM, 2.5 μM, 5 μM, and 10 μM, it can be confirmed that the fat globule staining was at a lower level compared to that of PA in a drug concentration dependent manner. In particular, even in comparison with PF-06409577 and elafibranor, which are substances already under development, it can be confirmed that the staining of fat globules was reduced not only at the same concentration of 10 μM but also at concentrations lower than the same.

These results show that the compound of the present invention not only has the effect of reducing fat globules, but also has an excellent effect of reducing fat accumulation in steatosis-induced liver cells compared to existing materials.

**Experimental Example 5. Verification of the expression of fat-oxidation promoting protein by the compound of the present invention compared to existing developed materials**

**Experimental Example 5.1. Experimental Method**

To induce steatosis in Huh-7 cells in each well except for control cells, the Huh-7 cells were treated with PA bound to 2% BSA at a concentration of 250 μM except for the control, and the control cells were pretreated with 2% BSA without FA.

The cells in each well were treated with the compounds of the present invention, BW-3290, 0.5 μM, 2.5 μM, and 10 μM, PF-06409577 1 μM and 10 μM, and elafibranor 1 μM and 10 μM, respectively and then cultured for 24 hours. The control cells and PA treated cells were treated with DMSO. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), aprotinin (2 μg/mL), 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected, 5X sodium dodecyl sulfate (SDS) sample buffer containing 50 mM Tris-Cl (pH 6.8), 2% SDS, 0.1% bromopheol blue, and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membrane of P-ACC and LCAD was washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

### Experimental Example 5.2. Experimental Results

FIG. 4 is a drawing showing the results of drug screening in steatosis-induced liver cells compared to existing materials under development for fat accumulation. That is, it is a drawing showing the expression level of the proteins associated with fat synthesis inhibition and fatty acid oxidation. Since ACC loses the ACC protein activity when phosphorylated, an increase in p-ACC represents a decrease in fat synthesis. Additionally, it can be confirmed that although the compound of the present invention, BW-3290, has a lower concentration than PF-064099577 and elafibranor, it has the same or higher effect of inhibiting fat accumulation in a concentration-dependent manner. Further, it could be confirmed that with regard to the expression of LCAD *(i.e.,* a fatty acid oxidation-related protein), the compound of the present invention, BW-3290, has the same or higher effect of fatty acid degradation in a concentration-dependent manner compared to PF-064099577 and elafibranor. From these results, it can be seen that the compound of the present invention has an improved effect of inhibiting fat accumulation compared to PF-064099577 and elafibranor.

### Experimental Example 6. Confirmation of effect of inhibiting fat accumulation in adipocytes (3T3L1 cells)

### Experimental Example 6.1. Experimental Method

Preparation of 3TL-L1 adipocyte progenitor cell and induction of differentiation

In order to confirm the effect of the compound on reducing fat accumulation on adipocytes, 3TL-L1 mouse fat progenitor cells (American Type Culture Collection) were cultured in an incubator set at 37°C with 95% humidity and 5% $CO_2$.

Additionally, after sufficiently proliferating 3T3-L1 mouse adipocytes using a proliferation medium (Dulbecco's modified Eagle's medium (DMEM) supplemented with 10% bovine serum (BS), 100 U/mL of penicillin, and 100 μg/mL of streptomycin), the cells were treated with a differentiation medium (DMEM containing 10% FBS, 0.5 mM 3-isobutyl-1-methylxanthine, 1 mM dexamethasone, and 5 μg/mL insulin) for 2 days.

Thereafter, the cells were treated with an induction medium (DMEM containing 10% FBS and 5 μg/mL insulin) for 2 days, the maintenance medium (DMEM containing 10% FBS) was replaced daily to maintain the differentiated adipocytes until the end of the experiment.

Confirmation of fat globules through Oil-Red-O staining

According to an embodiment of the present invention, in order to confirm the ability of the compound of Formula 2 to inhibit fat globule formation, the 3T3-L1 cells (which are fat progenitor cells) were induced to be differentiated into adipocytes, and then stained through Oil-Red-O staining.

First, 3T3-L1 cells (which are fat progenitor cells) were cultured in a 6 well plate with a diameter of 6 $cm^2$, except for the undifferentiation plate and differentiation, the test materials were diluted with a culture medium and then treated with 0.5 μM, 2.5 μM, 10 μM, and 20 μM.

Additionally, after removing the cell supernatant from the wells for Oil-Red-O staining, the washing process was performed twice with PBS.

Additionally, after washing, the resultant was fixed with 4% paraformaldehyde for 30 minutes.

Additionally, then, 60% 1,2-propanediol dehydration solution was added thereto and the mixture was incubated for 5 minutes.

Additionally, 5 minutes thereafter, the solution was removed and mix with Oil-Red-O coloring reagent (the ORO stock solution and distilled water were mixed at a ratio of 6 mL to 4 mL, and the mixture was filtered with a 0.45 μm filter to prepare the ORO solution) was added thereto to stain fat globules for 30 minutes.

Additionally, after staining, the remaining color reagent was removed by washing with PBS, and the formation of fat globules was observed under a microscope.

### Experimental Example 6.2. Experiment results

The experimental results confirming the effect of reducing fat accumulation in adipocytes are shown in FIGS. 5 and 6. In particular, FIGS. 5 and 6 are drawings showing the experimental results confirming the inhibition of fat globule formation of the compound through Oil-Red-O staining.

As shown in FIG. 5, it can be confirmed that the compound of the present invention has a lower level of fat globule staining compared to differentiation in all test groups. In particular, it can be seen that as a result of the experiment by increasing the concentration of the compound to 0.5 μM, 2.5 μM, 10 μM, and 20 μM, the compound showed a lower level of fat globule staining compared to differentiation in a drug concentration-dependent manner.

Additionally, as shown in FIG. 6, it can be confirmed that as the concentration of the compound of the present invention was increased to 0.5 μM, 2.5 μM, 10 μM, and 20 μM, the amount of stained fat globules appeared less in a drug concentration-dependent manner.

These results show that the compound of the present invention has an effect of reducing fat accumulation in adipocytes.

**Experimental Example 7. Confirmation of fat globules reducing effect in steatosis-induced hepatocytes (Huh7 cells)**

**Experimental Example 7.1. Experimental Method**

Preparation of Huh7 Cells

According to an embodiment of the present invention, in order to confirm the effect of inhibiting the accumulation of triglycerides in steatosis-induced liver cells, the Huh7 cells were cultured in an incubator set at 37°C with 95% humidity and 5% $CO_2$.

Additionally, Huh7 cells were proliferated to a confluency of 80-90% on a plate using a growth medium(Dulbecco ' s modified Eagle ' s medium (DMEM) supplemented with 10% fetal bovine serum (FBS), 100 U/ml of penicillin, and 100 μg/ml of streptomycin), and then used for the experiment.

Confirmation of fat globule reducing effect in steatosis-induced Huh7 cells through Oil-Red-O staining

According to an embodiment of the present invention, in order to confirm the ability of BW-3290 to inhibit fat globule accumulation in steatosis-induced liver cells, the liver cells were treated with palmitic acid (PA) bound to 2% BSA at a concentration of 250 μM except for the control group to induce steatosis, and then fat globules were stained through Oil-Red-O staining.

First, liver cells were cultured to grow 80-90% confluency in a 12 well plate, and the test material were diluted in the culture medium that induced steatosis and the liver cells, except for the control group, were treated with 0.5 μM, 2.5 μM, and 10 μM of the test material.

Additionally, after removing the cell supernatant from the wells for Oil-Red-O staining, the washing process was performed twice with PBS.

Additionally, after washing, the resultant was fixed with 4% paraformaldehyde for 30 minutes.

Additionally, then, 60% 1,2-propanediol dehydration solution was added thereto and the mixture was incubated for 5 minutes.

Additionally, 5 minutes thereafter, the solution was removed and mix with Oil-Red-O coloring reagent (the ORO stock solution and distilled water were mixed at a ratio of 6 mL to 4 mL, and the mixture was filtered with a 0.45 μm filter to prepare the ORO solution) was added thereto to stain fat globules for 30 minutes.

Additionally, after staining, the remaining color reagent was removed by washing with PBS, and the formation of fat globules was observed under a microscope.

**Experimental Example 7.2. Experiment results**

The experimental results confirming the effect of reducing fat accumulation in steatosis-induced Huh7 cells are shown in FIGS. 7 and 8. In particular, FIG. 7 is drawings showing the experimental results confirming the inhibition of fat globule formation of BW-3290 through Oil-Red-O staining. However, for the convenience of the experiment, the experimental results are presented only for the cells shown in FIG. 7, and the experimental results confirming the effect of reducing fat accumulation in adipocytes are not limited to the cells shown in FIG. 7, and the effect of reducing fat accumulation in adipocytes can also be confirmed for cells corresponding to cells inducing liver disease.

As shown in FIG. 7, it can be confirmed that the compound of the present invention has a lower level of fat globule staining compared to PA in all test groups. Considering that the experiment was performed by increasing the concentration of the compound to 0.5 μM, 2.5 μM, and 10 μM, it can be confirmed that the fat globule staining was at a lower level compared to that of PA in a drug concentration dependent manner

These results show that the compound of the present invention has the effect of reducing fat globules in steatosis-induced liver cells.

**Experimental Example 8. Confirmation of changes in expression of lipogenesis-related factors in steatosis-induced hepatocytes (Huh7 cells)**

**Experimental Example 8.1. Experimental Method**

Confirmation of expression level of proteins in steatosis-induced Huh-7 cells

To induce steatosis in Huh-7 cells in each well except for control cells, the Huh-7 cells were treated with PA bound to 2% BSA at a concentration of 250 μM except for the control, and the control cells were pretreated with 2% BSA without FA.

The cells in each well were treated with the compound of Chemical Formula 2 0.5 μM, 2.5 μM, and 10 μM and then cultured for 24 hours. The control cells and PA treated cells were treated with DMSO. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), 2 μg/mL aprotinin, 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected, 5X sodium dodecyl sulfate (SDS) sample buffer containing 50 mM Tris-Cl (pH 6.8), 2% SDS, 0.1% bromopheol blue, and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membranes of sterol regulatory element-binding protein 1 (SREBP1) and fatty acid synthase (FAS) were washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

Experimental Method for confirming results of quantitative analysis of mRNA in steatosis-induced Huh7 cells

To induce steatosis in Huh-7 cells in each well except for control cells, the Huh-7 cells were treated with PA bound to 2% BSA at a concentration of 250 μM except for the control, and the control cells were pretreated with 2% BSA without FA.

The cells in each well were treated with the compound of Chemical Formula 2 according to the present invention 0.5 μM and then cultured for 24 hours. The control cells and PA treated cells were treated with DMSO. All media were aspirated and the cells were gently washed 3 times with cold PBS.

Additionally, the total RNA was extracted using the RNeasy RNA isolation kit (Qiagen, Hilden, Germany).

Additionally, 10 μL of β per mL of a cell lysis solution was additionally prepared.

Additionally, the cells were washed twice with cold PBS and 350 μL of RNA lysis buffer was added thereto.

Additionally, 70% EtOH was added to the lysate in an equal volume, mixed by pipetting, transferred to a spin column, and centrifuged at 8,000x g for 15 seconds.

Additionally, the flow-through solution was removed and 700 μL of a wash solution was added to the spin column, centrifuged at 8,000x g, and the flow-through solution was removed.

Additionally, 500 μL of the wash buffer containing EtOH was added to the spin column and centrifuged at 8,000x g for 2 minutes (repeated 2 times)

Additionally, the flow-through solution was discarded and centrifuged once more to completely dry the spin column membrane.

Additionally, the spin column was placed into a new Eppendorf tube and 50 μL of RNase-free water was added directly to the spin column membrane and centrifuged at 8,000x g for 1 minute to dissolve and isolate the RNA.

Additionally, RNA concentration was measured at OD 260 nm using the Nanophotometer N60 (Implen, Munich, Germany)

Additionally, 1 μg of total RNA was reverse transcribed in the first strand synthesis buffer containing oligo (dT) primer (6 μg/mL), random primer (3 μg/mL), 50 U of reverse transcriptase, 4 mM dNTP, 5 mM dNTP, 5 mM magnesium chloride ($MgCl_2$), and 40 U of RNase inhibitor

Additionally, cDNA was synthesized using a polymerase chain reaction (PCR) device which was set to undergo denaturation at 95°C for 5 minutes, cDNA synthesis at 42°C for 50 minutes, and denaturation at 95°C for 5 minutes.

Additionally, the synthesized cDNA was diluted 1/10 and qRT-PCR was performed for the gene using the StepOnePlus Real-Time PCR System (Applied Biosystems, Foster City, CA, USA). (All of the primers for qRT-PCR were synthesized by GenoTech (Daejeon).)

Additionally, 3 μL of cDNA, 10 μL of Cyber Green (SYBR) master mix (Applied Biosystems), 3 μL of a primer mix (a sense primer and an antisense primer), and 4 μL of distilled water (DW) were mixed to prepare a reaction mixture, and the mixture was set to undergo 40 cycles of denaturation at 95°C for 15 seconds and annealing synthesis at 60°C for 1 minute, and synthesis were subjected to 40 cycles.

**Experimental Example 8.2. Experimental Results**

Experimental results confirming the protein expression level in steatosis-induced Huh7 cells are shown in FIG. 9-A.

This shows the results confirming the protein expression level for SREBP1 and FAS involved in fat synthesis with regard to Huh7 cells.

According to FIG. 9-A, it shown that as the concentration of the compound of BW-3290 was increased to 0.5 μM, 2.5 μM, and 10 μM, the level of SREBP1 and FAS decreased in a concentration-dependent manner.

Experimental results confirming the results of quantitative analysis of mRNA in steatosis-induced Huh7 cells are shown in FIG. 9-B

This shows the results of quantitative analysis of mRNA related to gene expression of SREBP-1c and FAS involved in fat synthesis.

Referring to FIG. 9-B, it can be seen that BW-3290 reduced the gene expression of SREBP-1c and FAS involved in fat synthesis compared to the control group.

Accordingly, it is shown that the compound of the present invention, BW-3290, reduces the expression of fat synthesis proteins and genes.

**Experimental Example 9. Confirmation of changes in expression of fatty acid beta oxidation factor in steatosis-induced hepatocytes (Huh7 cells)**

**Experimental Example 9.1. Experimental Method**

To induce steatosis in Huh-7 cells in each well except for control cells, the Huh-7 cells were treated with PA bound to 2% BSA at a concentration of 250 μM except for the control, and the control cells were pretreated with 2% BSA without FA.

The cells in each well were treated with the compound of Chemical Formula 2 0.5 μM, 2.5 μM, and 10 μM and then cultured for 24 hours. The control cells and PA treated cells were treated with DMSO. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), 2 μg/mL aprotinin, 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected, 5X sodium dodecyl sulfate (SDS) sample buffer containing 50 mM Tris-Cl (pH 6.8), 2% SDS, 0.1% bromopheol blue,

and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membranes of SIRT3, PGC1α, PPARα, and LCAD were washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

**Experimental Example 9.2. Experimental Results**

Experimental results confirming the expression level of proteins related to fat oxidation in steatosis-induced Huh7 cells are shown in FIG. 10.

FIG. 10 shows the results confirming the protein expression levels for SIRT3, PPARα, PGC1α, and LCAD involved in fatty acid beta (β) oxidation with regard to Huh7 cells.

It can be confirmed that as the concentration of BW-3290, the compound of the present invention, increased to 0.5 μM, 2.5 μM, and 10 μM, the amount of SIRT3, PPARα, PGC1α, and LCAD, which are proteins involved in fatty acid oxidation in a concentration-dependent manner.

**Experimental Example 10. Confirmation of changes in expression of inflammatory factors in steatosis-induced hepatocytes (Huh7cells)**

**Experimental Example 10.1. Experimental Method**

<u>Confirmation of protein expression levels in liver cells induced with inflammatory response</u>

To induce steatosis in Huh-7 cells in each well except for control cells, the Huh-7 cells were pretreated with 10ng/ml IL-1β for 2 hr except for the control.

The cells in each well were treated with the compound of the present invention, BW-3290, 0.5 μM, 2.5 μM, and 10 μM and then cultured for 24 hours. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), 2 μg/mL aprotinin, 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected, 5X sodium dodecyl sulfate (SDS) sample buffer containing 50 mM Tris-Cl (pH 6.8), 2% SDS, 0.1% bromopheol blue, and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The

gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membranes of p-NPκ and p-IKKα were washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

<u>Confirmation of gene expression in liver cells induced with inflammatory response</u>

After culturing the Huh-7 cells to grow to a confluency of 80-90% in a 6 well plate, except for the control, the cells were pretreated with IL-1β and then treated with BW-3290, which is the compound of the present invention, diluted in the culture medium to concentrations of 0.5 μM, 2.5 μM, and 10 μM, so as to induce an inflammatory response.

Additionally, the total RNA was extracted using the RNeasy RNA isolation kit (Qiagen, Hilden, Germany).

Additionally, 10 μL of β per mL of a cell lysis solution was additionally prepared.

Additionally, the cells were washed twice with cold PBS and 350 μL of RNA lysis buffer was added thereto.

Additionally, 70% EtOH was added to the lysate in an equal volume, mixed by pipetting, transferred to a spin column, and centrifuged at 8,000x g for 15 seconds.

Additionally, the flow-through solution was removed and 700 μL of a wash solution was added to the spin column, centrifuged at 8,000x g, and the flow-through solution was removed.

Additionally, 500 μL of the wash buffer containing EtOH was added to the spin column and centrifuged at 8,000x g for 2 minutes (repeated 2 times)

Additionally, the flow-through solution was discarded and centrifuged once more to completely dry the spin column membrane.

Additionally, the spin column was placed into a new Eppendorf tube and 50 μL of RNase-free water was added directly to the spin column membrane and centrifuged at 8,000x g for 1 minute to dissolve and isolate the RNA.

Additionally, RNA concentration was measured at OD 260 nm using the Nanophotometer N60 (Implen, Munich, Germany)

Additionally, 1 μg of total RNA was reverse transcribed in the first strand synthesis buffer containing oligo (dT) primer (6 μg/mL), random primer (3 μg/mL), 50 U of reverse transcriptase, 4 mM dNTP, 5 mM dNTP, 5 mM magnesium chloride ($MgCl_2$), and 40 U of RNase inhibitor.

Additionally, cDNA was synthesized using a polymerase chain reaction (PCR) device which was set to undergo denaturation at 95°C for 5 minutes, cDNA synthesis at 42°C for 50 minutes, and denaturation at 95°C for 5 minutes.

Additionally, the synthesized cDNA was diluted 1/10 and qRT-PCR was performed for the gene using the StepOnePlus Real-Time PCR System (Applied Biosystems, Foster City, CA, USA). (All of the primers for qRT-PCR were synthesized by GenoTech (Daejeon).)

Additionally, 3 μL of cDNA, 10 μL of Cyber Green (SYBR) master mix (Applied Biosystems), 3 μL of a primer mix (a sense primer and an antisense primer), and 4 μL of distilled water (DW) were mixed to prepare a reaction mixture, and the mixture was set to undergo 40 cycles of denaturation at 95°C for 15 seconds and annealing synthesis at 60°C for 1 minute, and synthesis were subjected to 40 cycles.

**Experimental Example 10.2. Experimental Results**

Experimental results confirming the expression levels of proteins and genes in Huh7 cells induced with inflammatory response are shown in FIG. 11 and FIG. 12.

FIG. 11 shows the results confirming the protein expression levels of p-NFκ and p-IKKα, and the gene expression levels of IL-6, 8, and TNF-α involved in the inflammatory response with regard to Huh7 cells.

It was confirmed that as the concentration of the compound of the present invention, BW-3290, increased to

0.5 μM, 2.5 μM, and 10 μM, the expression of proteins and genes involved in the inflammatory response decreased in a concentration-dependent manner.

It was confirmed that when the genes of TNFα and IL-8, which are inflammatory genes known as the cause of NASH, were identified, respectively, the expression levels of the genes of TNFa and IL-8 were each decreased, contrary to the control group.

Through these, it can be seen that the compound of the present invention has a therapeutic effect even on fatty liver-related diseases such as NASH.

**Experimental Example 11. Verification of activity as a multiple mode regulator**

**Experimental Example 11.1. Experimental Method**

Measurement of SIRT1 activity

All of the materials were provided from Sirt1 direct fluorescent screening assay kit (Cayman, Ann Arbor, MI, USA).

SIRT1, p53 conjugated aminomethylcoumarin (AMC) protein, and 3 mM NAD+ were added to diluted assay buffer (50 mM Tris-HCl, pH 8.0, 137 mM NaCl, 2.7 mM KCl, and 1 mM MgCh).

Three 100% initial activity wells among the 96-wells were added with 25 μL of assay buffer, 5 μL of diluted SIRT1, and 5 μL of a solvent.

Three background wells among the 96-wells were added with 30 μL of assay buffer and 5 μL of a solvent.

Three sample wells among the 96-wells were added with 25 μL of assay buffer, 5 μL of diluted SIRT1, and 5 μL of a diluted test compound. The final DMSO concentration was lower than 2%.

The reaction was started by adding 15 μL of a substrate solution containing 3 mM NAD+ to all of the wells to be used. The 96 wells were covered with a plate cover and incubated on a shaker at room temperature (25°C to 30 °C) for 45 minutes.

A reaction stop/developing solution was prepared. To prepare the final 5 mL solution, add 200 μL of nicotinamide (NAM, Sigma-Aldrich) was added to color powder weighed as 30 mg, 4.8 mL of diluted assay buffer was added and the mixture was stirred until the color powder became a solution.

After removing the plate cover, 50 μL of the stop/developing solution was added to each well.

The 96 wells were covered with a plate cover and incubated at room temperature (25°C to 30°C) for 30 minutes.

After removing the plate cover, the plate was read with Cytation 5 using an excitation wavelength at 360 nm and an emission wavelength at 450 nm.

Then, the average level of fluorescence for each sample was measured.

The fluorescence level of the background well was subtracted from the fluorescence level of the 100% starting active well and that of the sample well.

To determine the percentage of each sample, each sample value was subtracted from the 100% initial activity value, divided by the 100% initial activity value, and multiplied by 100, and the resulting value was added to 100 and expressed as a percentage.

$$\text{SIRT1 activation level of a sample (\%)} = 100 + (\text{initial activity value} - \text{sample value})/(\text{initial activity value}) \times 100$$

Experimental Method (Treatment of AMPK inhibitor)

To induce steatosis in Huh-7 cells in each well except for control cells, the Huh-7 cells were treated with PA bound to 2% BSA at a concentration of 250 μM except for the control, and the control cells were pretreated with 2% BSA without FA. To prove the *in vitro* activity of AMPK for confirming the activity of a multiple mode regulator by BW-3290, the AMPK was treated with 10 μM of compound C *(i.e.,* an AMPK inhibitor).

The cells in each well were treated with 10 μM and 20 μM BW-3290, and then cultured for 24 hours. The control cells and PA treated cells were treated with DMSO. All media were aspirated and the cells were gently washed 3 times with cold PBS.

A cold lysis buffer containing 150 mM sodium chloride (NaCl), 5 mM ethylenediaminetetraacetic acid (EDTA), pH 8.0, 10 mM 4-(2-hydroxyethyl)-1-piperazineethanesulfonic acid (HEPES), 1% nonylphenoxypolyethoxylethanol (NP-40), 2 μg/mL aprotinin, 1 μM pepstatin A, 10 μM leupeptin, 1 mM sodium fluoride (NaF), 0.2 mM sodium orthovanadate ($Na_2VO_4$), and 1 μM phenylmethylsulfonyl fluoride (PMSF) was added thereto.

A whole cell lysate was collected and gently pipetted at least 10 times to ensure complete lysis. The sample was incubated on ice for 20 minutes and centrifuged 4°C at 13,000 rpm, for 20 minutes. The supernatant was collected,

5X sodium dodecyl sulfate (SDS) sample buffer containing 50 mM Tris-Cl (pH 6.8), 2% SDS, 0.1% bromopheol blue, and 10% glycerol was added to the sample, and boiled at 95°C for 5 minutes.

The wells of 10% SDS-polyacrylamide gel electrophoresis (SDS-PAGE) were loaded with the same amount of a protein along with the molecular weight marker. The gel was electrophoresed at 100 V for 30 minutes in an electrophoresis chamber (Bio-Rad Laboratories, Hercules, CA, USA).

A polyvinylidene fluoride (PVDF) membrane (MilliporeSigma, Burlington, MA, USA) was activated with methanol (MeOH) for 1 minute and washed with a transfer buffer containing 25 mM Tris, 190 mM glycine, and 20% MeOH. The gel was placed in the transfer buffer for 10 minutes.

The transfer sandwich (in the order of paper, gel, membrane, and paper) was assembled and it was checked whether no air bubbles were trapped in the transfer sandwich. The transfer sandwich was placed in a transfer tank (Bio-Rad Laboratories) and ice cubes were placed in the transfer tank to prevent the transfer buffer from being heated. The protein was transferred from the gel to the PVDF membrane at 100 V for 30 minutes.

After washing the PVDF membrane with distilled water, the membrane was blocked for 1 hour at room temperature with a blocking buffer prepared by 5% skim milk (Difco Laboratories, Franklin Lakes, NJ, USA) in TBST containing 0.1% tween 20 (TBST, Sigma-Aldrich).

The membrane was incubated in a buffer in which the primary antibody was appropriately diluted. The membranes of p-AMPK and p-ACC were washed 3 times with TBST for 5 minutes each.

The membrane was incubated with a recommended dilution of horseradish peroxidase (HRP)-conjugated secondary mouse antibody in blocking buffer at room temperature for 1 hour.

The membrane was washed 5 times with TBST for 5 minutes each. Excess reagent was removed and the membrane was covered with a clear plastic wrap.

ECL prime western blotting detection reagent (GE Healthcare, Chicago, IL, USA) was applied according to the manufacturer's recommendations.

Images were acquired using an ImageQuant LAS 3000 (Fujifilm, Tokyo, Japan). Data were analyzed.

**Experimental Example 11.2. Experimental Results**

Experimental results confirming the expression level of proteins for verification of a multiple mode regulator in steatosis-induced Huh7 cells are shown in FIG. 13, FIG. 14 and FIG. 15.

FIG. 13 shows the results of confirming the protein expression levels for SIRT1 and AMPK, which act in a multiple mode in Huh7 cells.

FIG. 14 shows the results of confirming the SIRT1 activity level of BW-3290. When compared with the control (RSV, resveratrol), which is well known as an SIRT1 activator, it can be seen that BW-3290 further increases the activity level of SIRT1. This shows that BW-3290 directly binds to SIRT1 and increases its activity.

FIG. 15 shows the results of confirming the protein expression levels for p-AMPK and p-ACC, which show the activity of AMPK, using an AMPK inhibitor with respect to AMPK activity in the multiple mode of BW-3290.

It can be confirmed that as the concentration of the compound of the present invention, BW-3290, increases to 0.5 μM, 2.5 μM, and 10 μM, the proteins involved in SIRT1 and AMPK applied to multiple modes increase in a concentration-dependent manner, the activity of SIRT1 increases, and even when AMPK activity is inhibited using an AMPK inhibitor, BW-3290 increases the activity of SIRT1 and AMPK and acts in a multiple mode.

**Experimental Example 12. PK identification by the compound of the present invention**

This experiment was performed by GVKBio by a request.

**Experimental Example 12.1. Experimental Method**

An experiment was performed to measure the pharmacokinetic parameters of BW-3290 using three male Sprague Dwaley (SD) rats.

As for BW-3290, a formulation with a composition of 95% water containing 5% DMSO and 20% HPbCD was used, and was orally administered at a concentration of 10 mg/kg.

Blood samples were collected from the jugular vein at time points of 0.25, 0.5, 1, 4, 8, 10, 12, and 24 hours.

The blood samples collected were centrifuged for 15 minutes at 4°C at 2,500 × g to secure plasma.

Bioanalysis for the measurement of pharmacokinetic parameters was analyzed by LC-MS/MS.

**Experimental Example 12.2. Experimental Results**

The pharmacokinetic parameters of 10 mg/kg of BW-3290 are shown in FIG. 16. It was confirmed that BW-3290 had excellent AUClast and Cmax, and its concentration was maintained to some extent even after 12 hours after absorption.

**Experimental Example 13. Confirmation of *in-vivo* activity of the compound of the present invention**

This Experimental Example was performed by SanyalBio.

**Experimental Example 13.1. Experimental Method**

The DIAMOND™ mouse model, an animal model prepared using a Western diet and sugar water, was used. The DIAMOND™ mice were induced with WDSW for 14 weeks, and 12 mice were used for the control group and 12 mice for the BW-3290 treatment group, and BW-3290 was orally administered at 16 mg/kg once daily for 8 weeks. An autopsy was performed at week 22, and blood collection and tissue storage were performed for the individuals in each group.

**Experimental Example 13.2. Experimental Results**

After completion of the test at week 22, body weight and liver weight were shown to be decreased in the BW-3290 group compared to the control group (VC) (FIG. 17). It was confirmed that the decrease in liver weight was slightly greater than that in body weight, which was confirmed to improve the liver/body weight ratio.

It was confirmed that alanine aminotransferase (ALT), aspartate aminotransferase (AST), and alkaline phosphatase (ALP), which are liver function enzymes that can confirm liver disease or liver damage, increased in the control group (VC), and the levels of all of these enzymes were decreased by BW-3290.

In particular, it was confirmed that ALT and AST, which are mainly expressed in the liver, showed statistical significance and their levels were decreased compared to those of the control group.

It can be confirmed that the compound of the present invention, BW-3290, shows the effect of reducing body weight and liver weight, that it reduces enzymes involved in liver function, and that it acts as a therapeutic agent for NASH in experiments using animal models.

**Experimental Example 14. Confirmation of *in-vivo* activity of the compound of the present invention (histopathological diagnosis)**

This Experimental Example was performed by SanyalBio.

**Experimental Example 14.1. Experimental Method**

Hematoxylin & Eosin (H&E) staining and Oil-Red-O (ORO) staining were performed using liver tissue obtained after autopsy

**Experimental Example 14.2. Experiment results**

H&E staining is a basic staining method in histology, and it could be confirmed that compared to the control group, the fat globules shown as a round matter in the image were reduced in the BW-3290 treatment group, which is the compound of the present invention (FIG. 18).

The level of staining of fat globules in liver tissue can be confirmed through ORO staining, and it could be confirmed that the fat cells stained in red were shown to be reduced in the BW-3290 treatment group, which is the compound of the present invention, compared to the control group (VC).

Therefore, it was confirmed that fat accumulation was reduced by BW-3290 by liver histological findings, and through this, it was confirmed in animal experiments that the compound of the present invention has a therapeutic effect on fatty liver-related diseases such as NASH.

**Experimental Example 15. Confirmation of efficacy in improving liver fibrosis**

This Experimental Example was performed by SanyalBio.

**Experimental Example 15.1. Experimental Method**

Staining was performed by Sirius Red staining and Trichrome (Masson's trichrome) staining, which are the

staining methods that can confirm fibrosis, was stained using the liver tissue obtained after autopsy.

**Experimental Example 15.2. Experimental Results**

The collagen and amyloid tissues stained in red by Sirius Red staining were shown to be decreased in the BW-3290 treatment group compared to the control group (VC) (FIGS. 19 and 22).

The fiberized portions stained in blue by Trichrome staining were reduced in the BW-3290 treatment group compared to the control group (VC).

Through this, it can be confirmed that the liver fibrosis in the liver tissue is improved by BW-3290, which is the compound of the present invention, and it can be confirmed through animal experiments that BW-3290 is effective in treating not only fatty liver and NASH, but also liver fibrosis.

**Experimental Example 16. Confirmation of changes in insulin resistance**

This Experimental Example was performed by SanyalBio.

**Experimental Example 16.1. Experimental Method**

The insulin resistance test was performed one week before autopsy, and the subject was fasted for 6 hours.

Insulin was injected intraperitoneally at a concentration of 0.75 UU/kg, and the blood was collected 15 minutes, 30 minutes, 45 minutes, and 90 minutes after the injection, and the blood glucose levels were measured using the OneTouch Plus Ultra Glucometer and glucose scores were evaluated therefrom.

**Experimental Example 16.2. Results**

It was confirmed that the glucose score was decreased in the BW-3290 group at 0 and 90 minutes showing statistical significance compared to the control group (VC) (FIG. 20).

Accordingly, BW-3290, the compound of the present invention, shows its potential to act as an insulin sensitizer as well as a therapeutic agent for NASH based on the Experimental Results of insulin resistance, thereby showing the possibility of expanding its indication to type 2 diabetes.

**Claims**

1. A compound of Chemical Formular 1 or a pharmaceutically acceptable salt thereof:

[Chemical Formular 1]

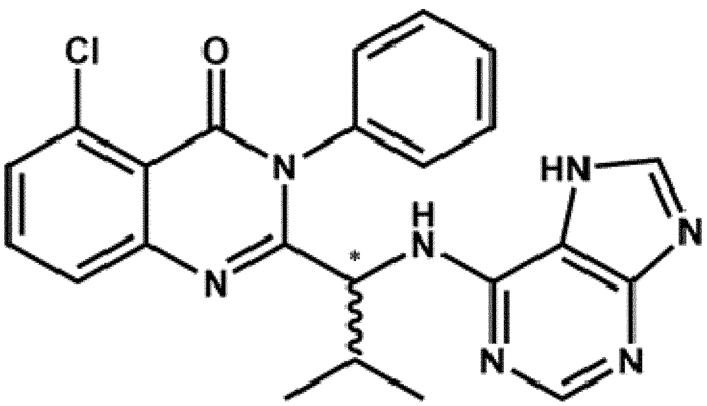

wherein * is a stereocenter, and the compound is an S-form or R-form based on the stereocenter, or a racemate in which the S-form and the R-form are mixed.

2. The compound or the pharmaceutically acceptable salt thereof according to claim 1, wherein the compound is selected from the compound of Chemical Formular 3 and the compound of Chemical Formular 4:

[Chemical Formular 3]

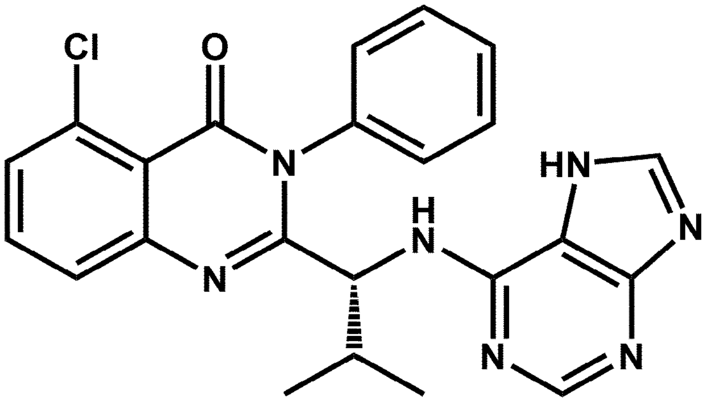

[Chemical Formular 4]

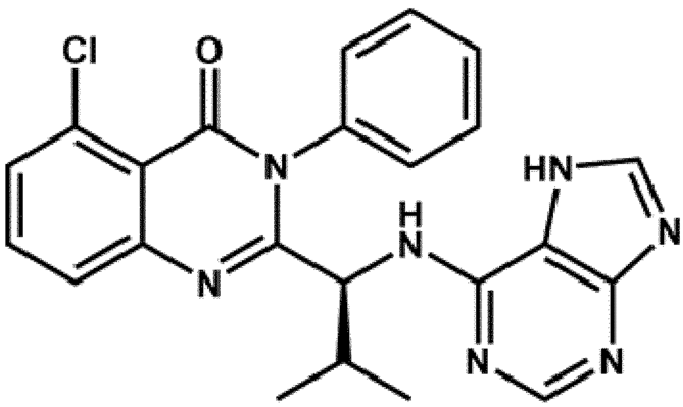

**3.** The compound or the pharmaceutically acceptable salt thereof according to claim 1, wherein the pharmaceutically acceptable salt is a hydrochloride salt.

**4.** A pharmaceutical composition for treating of a lipid metabolic disease, comprising the compound of Chemical Formular 1 or a pharmaceutically acceptable salt thereof:

[Chemical Formular 1]

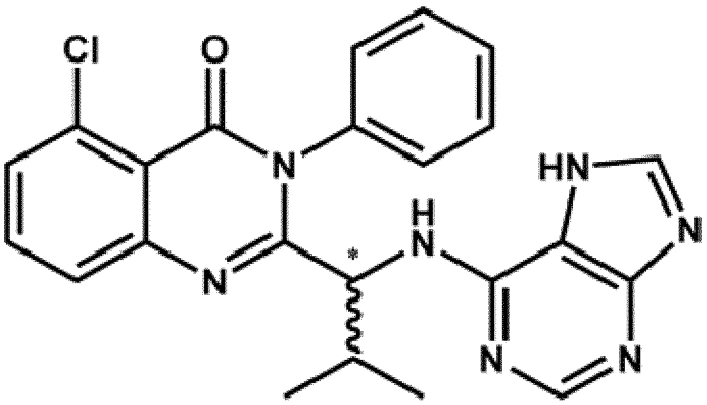

wherein * is a stereocenter, and the compound is an S-form or R-form based on the stereocenter, or a racemate in which the S-form and the R-form are mixed.

**5.** The pharmaceutical composition according to claim 4, wherein the lipid metabolic disease is selected from non-alcoholic fatty liver disease (NAFLD), non-alcoholic steatohepatitis (NASH), and liver fibrosis.

**6.** The pharmaceutical composition according to claim 4, wherein the compound is selected from the compound of Chemical Formular 3 and the compound of Chemical Formular 4:

[Chemical Formular 3]

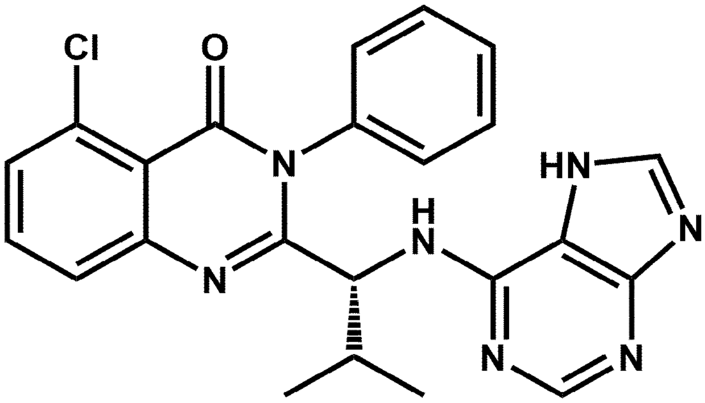

[Chemical Formular 4]

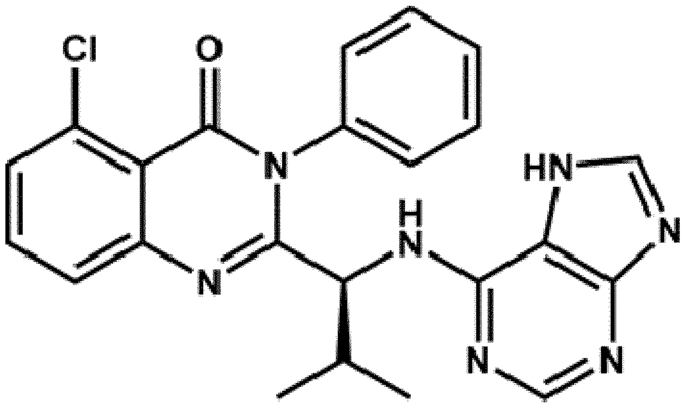

[FIG. 1]

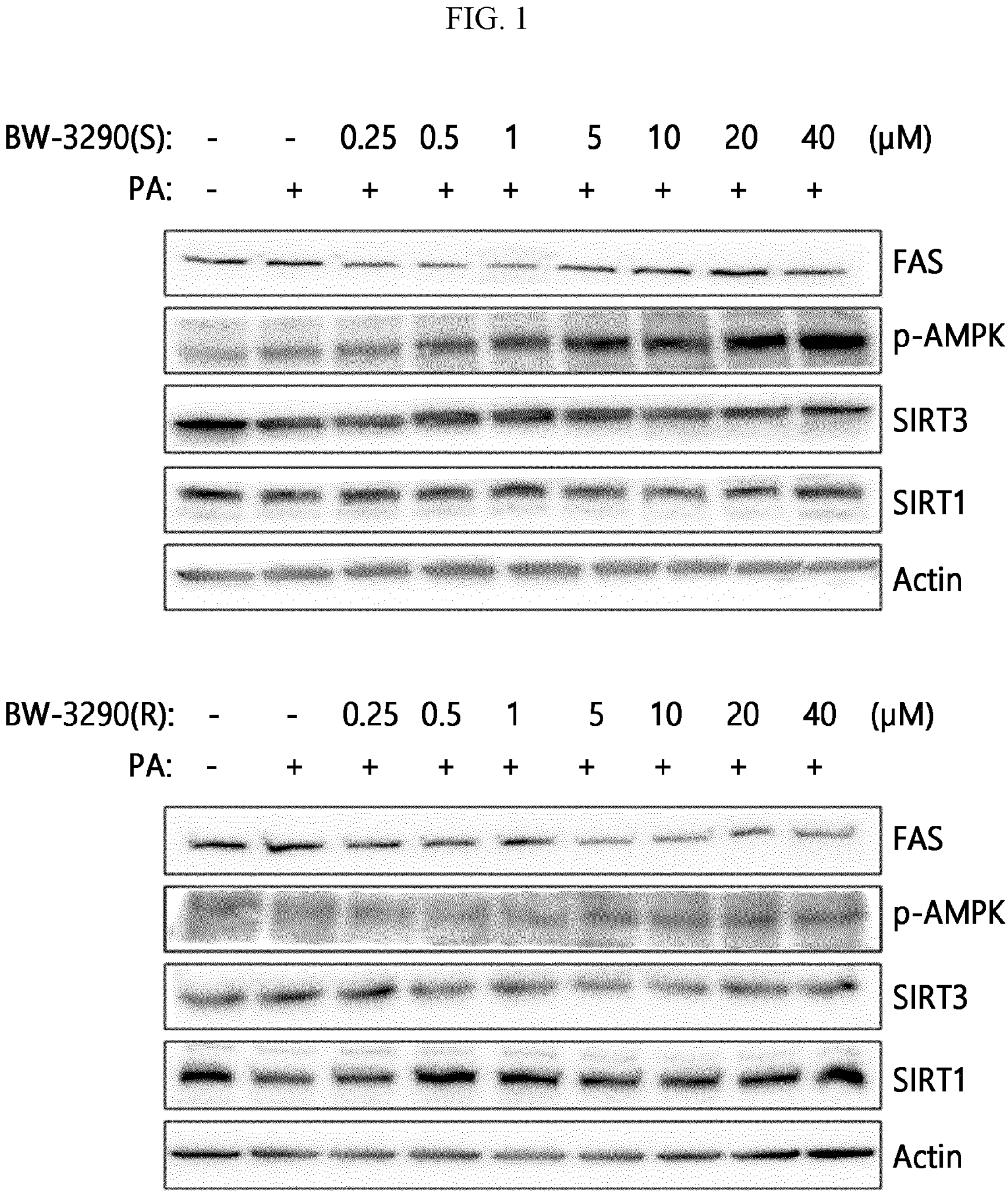
FIG. 1
BW-3290(S): - - 0.25 0.5 1 5 10 20 40 (μM)
PA: - + + + + + + + +
FAS
p-AMPK
SIRT3
SIRT1
Actin
BW-3290(R): - - 0.25 0.5 1 5 10 20 40 (μM)
PA: - + + + + + + + +
FAS
p-AMPK
SIRT3
SIRT1
Actin

[FIG. 2]

FIG. 2

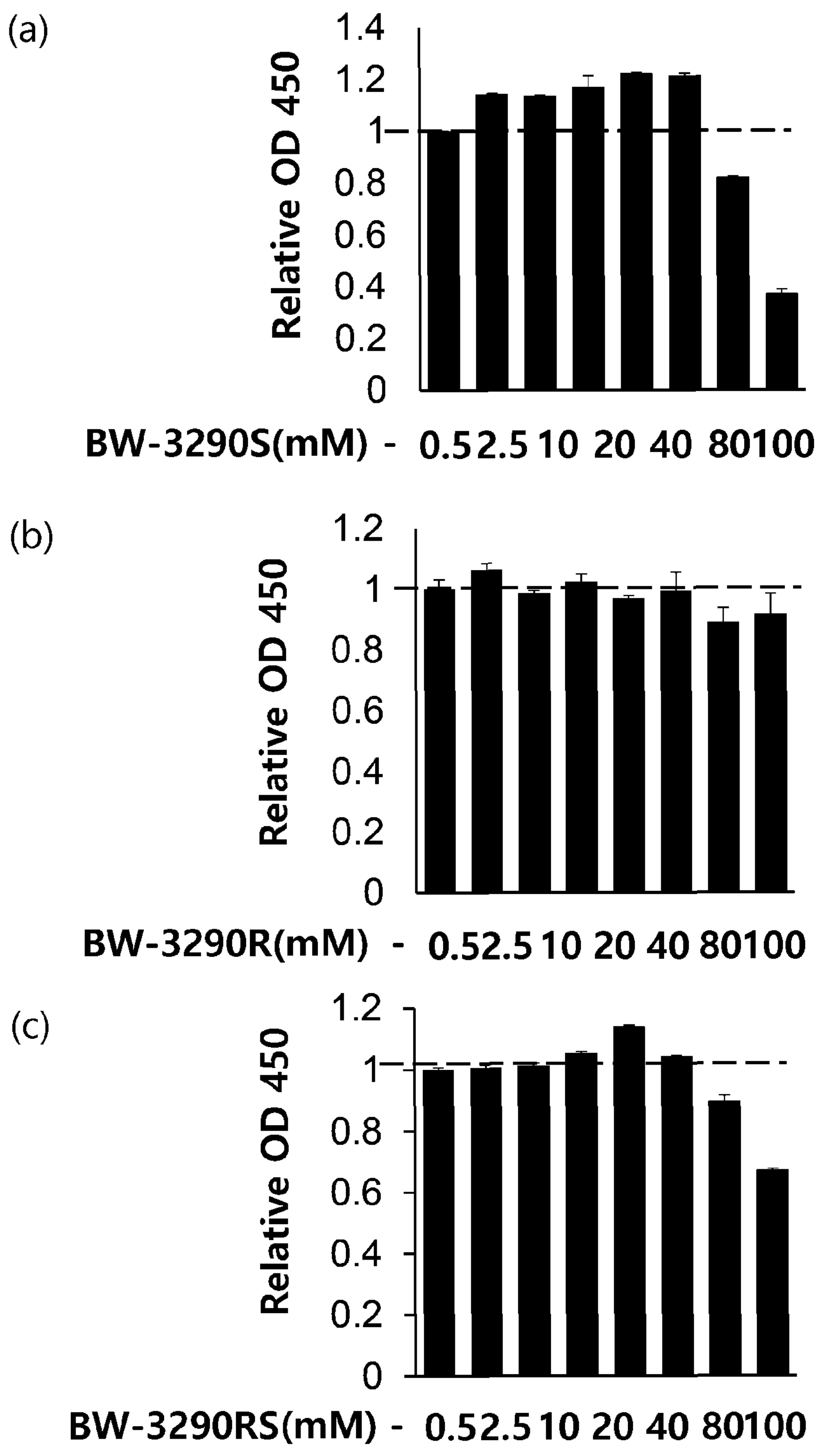
(a)
1.4
1.2
1
0.8
0.6
0.4
0.2
0
Relative OD 450
BW-3290S(mM) - 0.5 2.5 10 20 40 80 100
(b)
1.2
1
0.8
0.6
0.4
0.2
0
Relative OD 450
BW-3290R(mM) - 0.5 2.5 10 20 40 80 100
(c)
1.2
1
0.8
0.6
0.4
0.2
0
Relative OD 450
BW-3290RS(mM) - 0.5 2.5 10 20 40 80 100

[FIG. 3]

FIG. 3

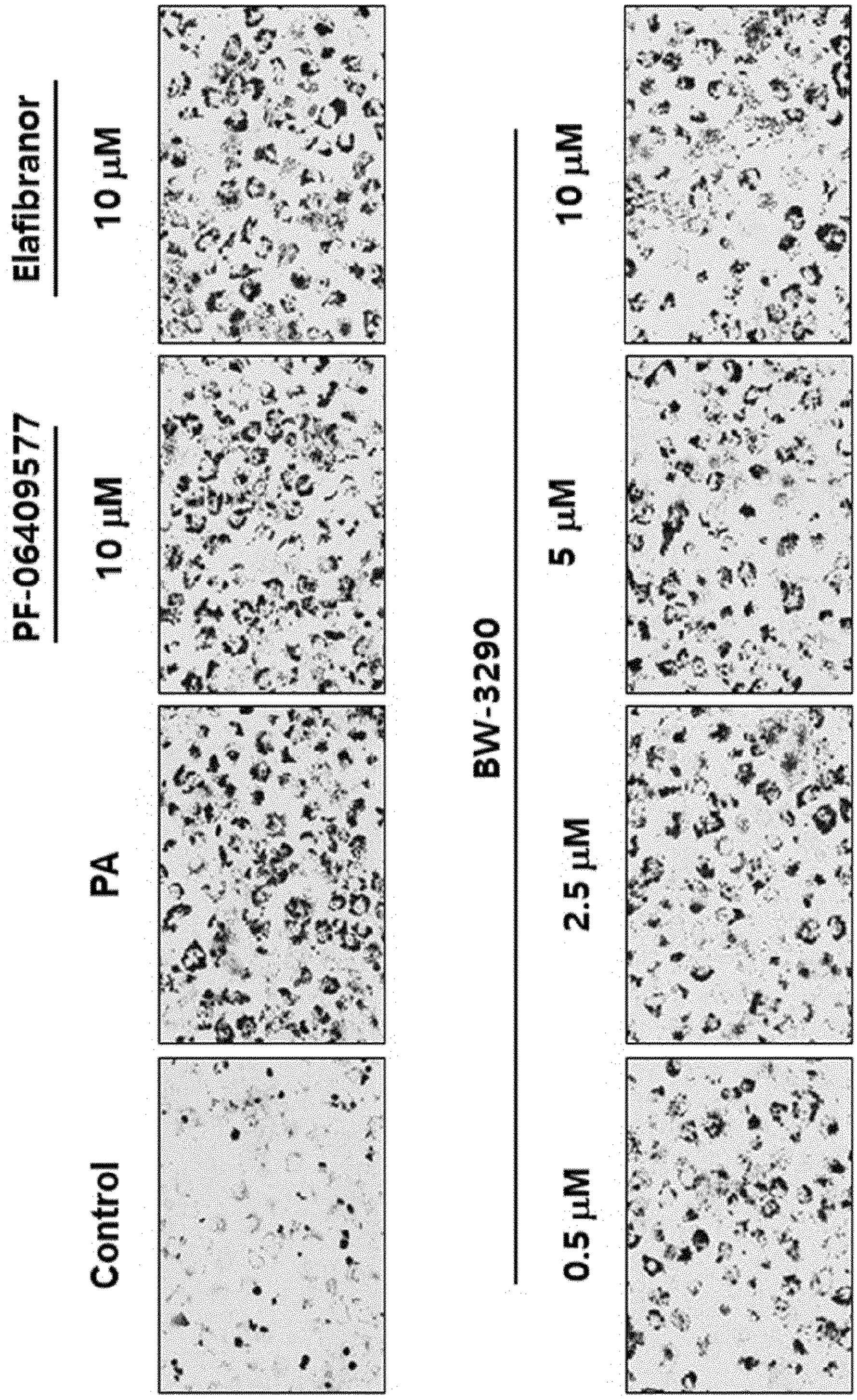
Control
PA
PF-06409577
10 μM
Elafibranor
10 μM
BW-3290
0.5 μM
2.5 μM
5 μM
10 μM

[FIG. 4]

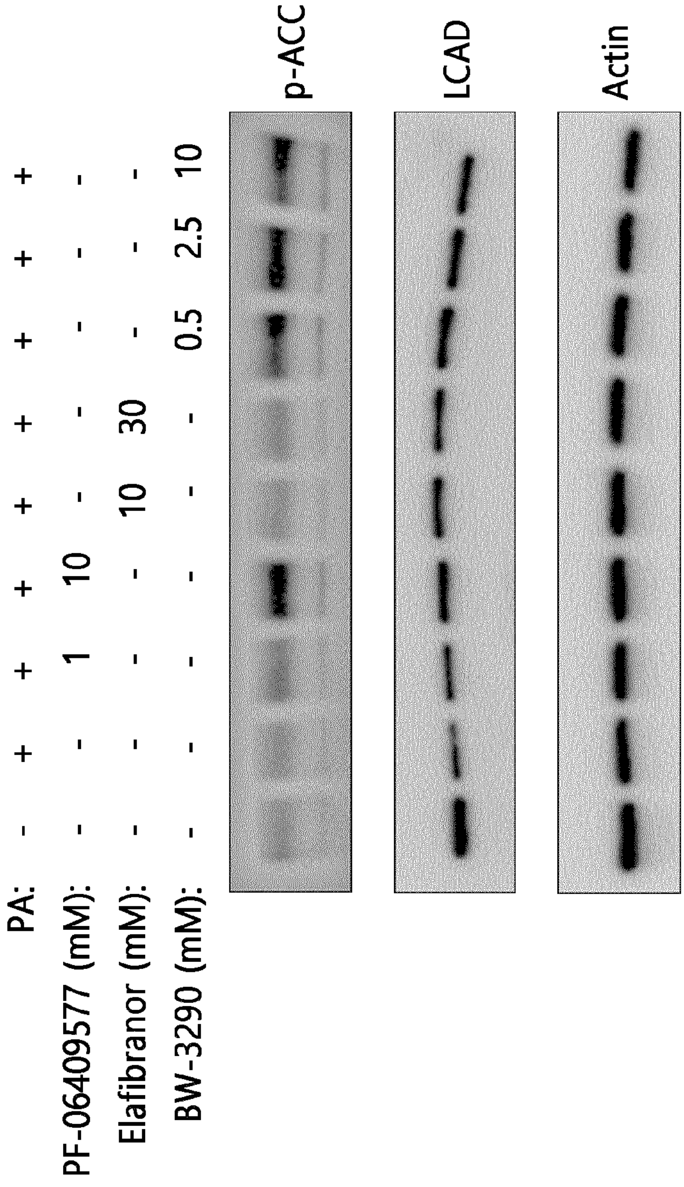
PA:
PF-06409577 (mM):
Elafibranor (mM):
BW-3290 (mM):
- + + + + + + + +
- - 1 10 - - - - -
- - - - 10 30 - - -
- - - - - - 0.5 2.5 10
p-ACC
LCAD
Actin

FIG. 4

[FIG. 5]

FIG. 5

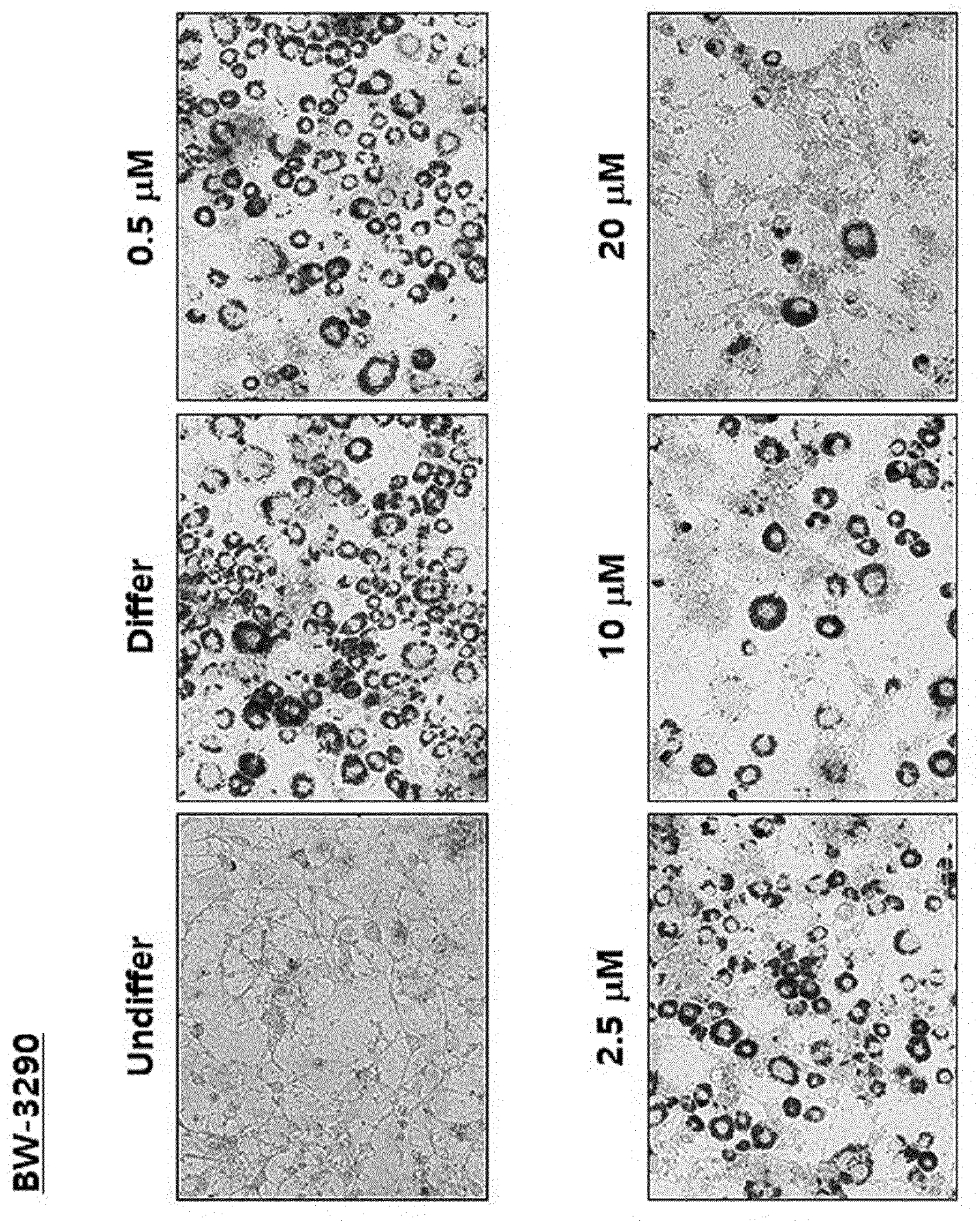

[FIG. 6]

FIG. 6

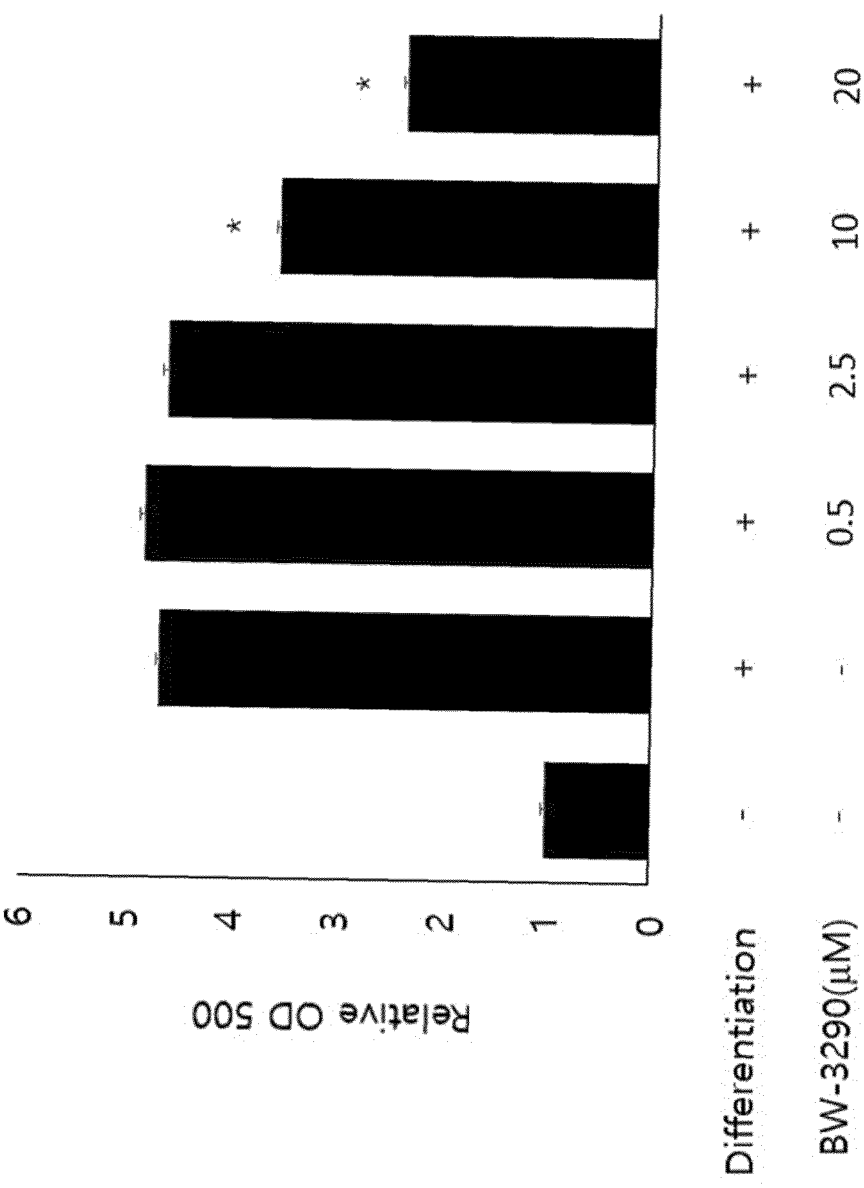
Relative OD 500
0
1
2
3
4
5
6
Differentiation
BW-3290(μM)
-
+
+
+
+
+
-
-
0.5
2.5
10
20
*
*

[FIG. 7]

FIG. 7

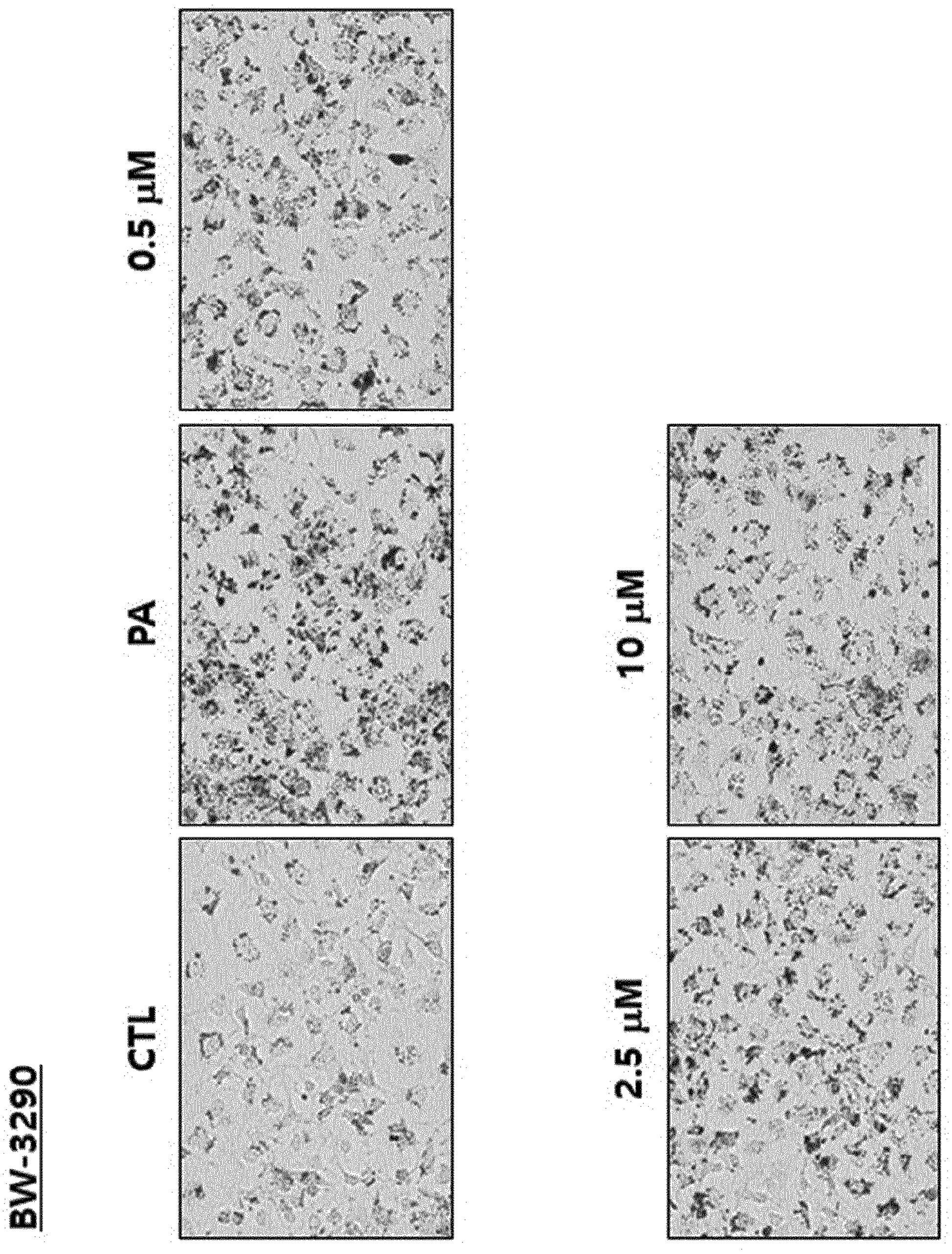

[FIG. 8]

FIG. 8

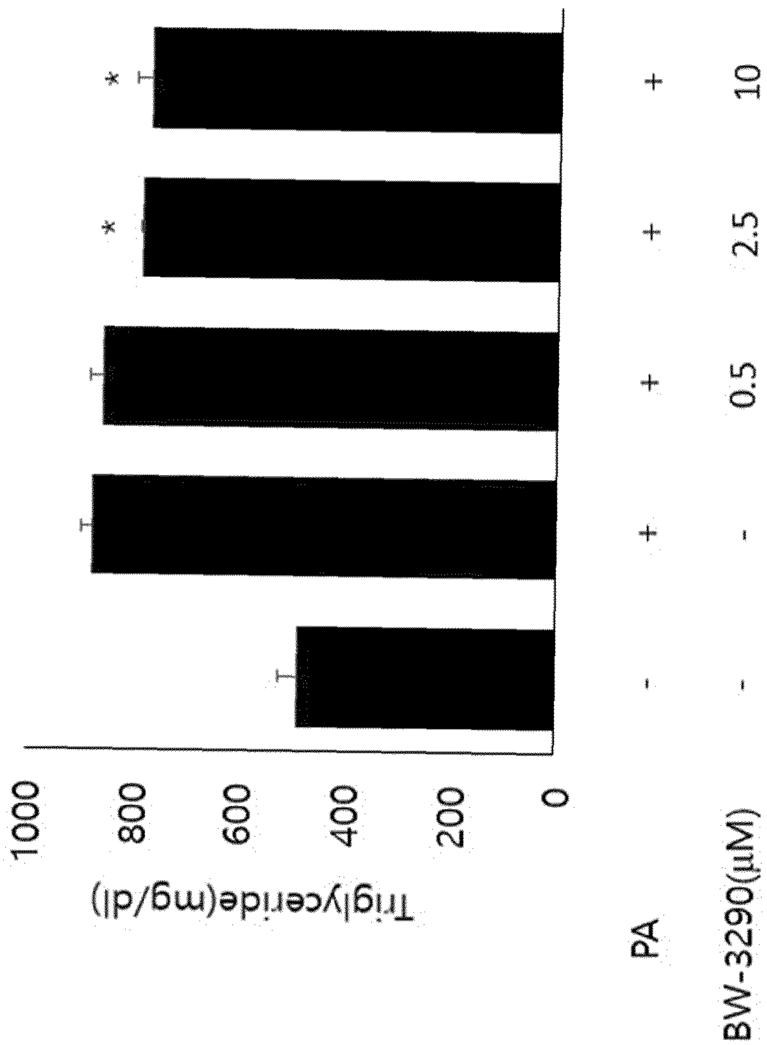

[FIG. 9]

FIG. 9

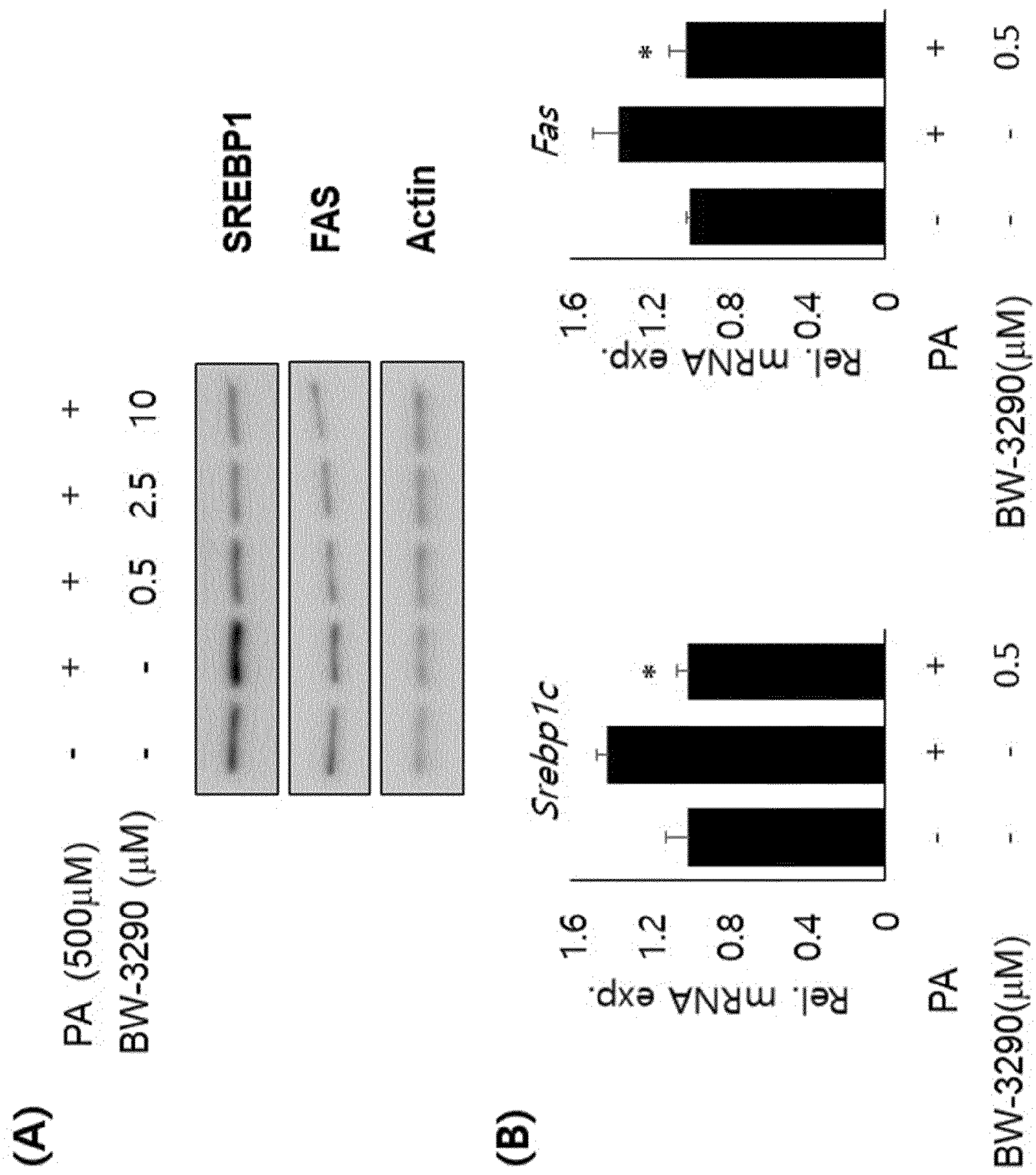

(A)
PA (500μM)
BW-3290 (μM)
- + + + +
- - 0.5 2.5 10
SREBP1
FAS
Actin
(B)
Srebp1c
Fas
Rel. mRNA exp.
1.6
1.2
0.8
0.4
0
*
PA
BW-3290(μM)
- + +
- - 0.5

[FIG. 10]

FIG. 10

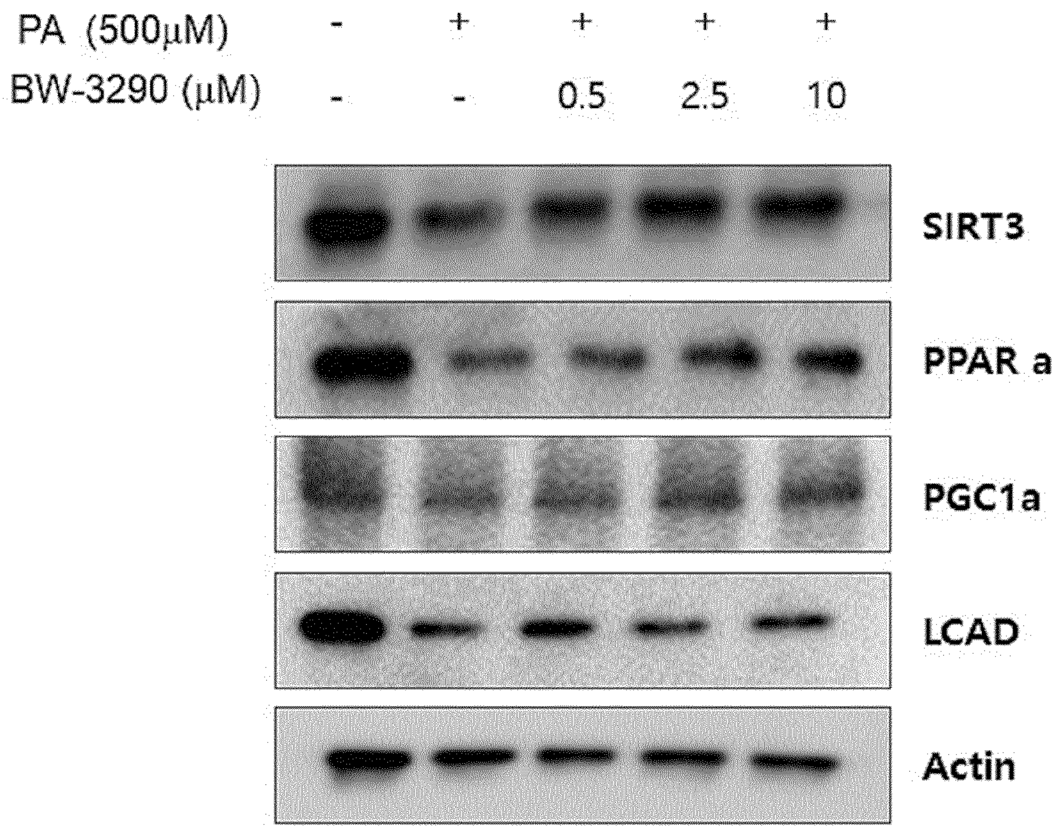

**[FIG. 11]**

FIG. 11

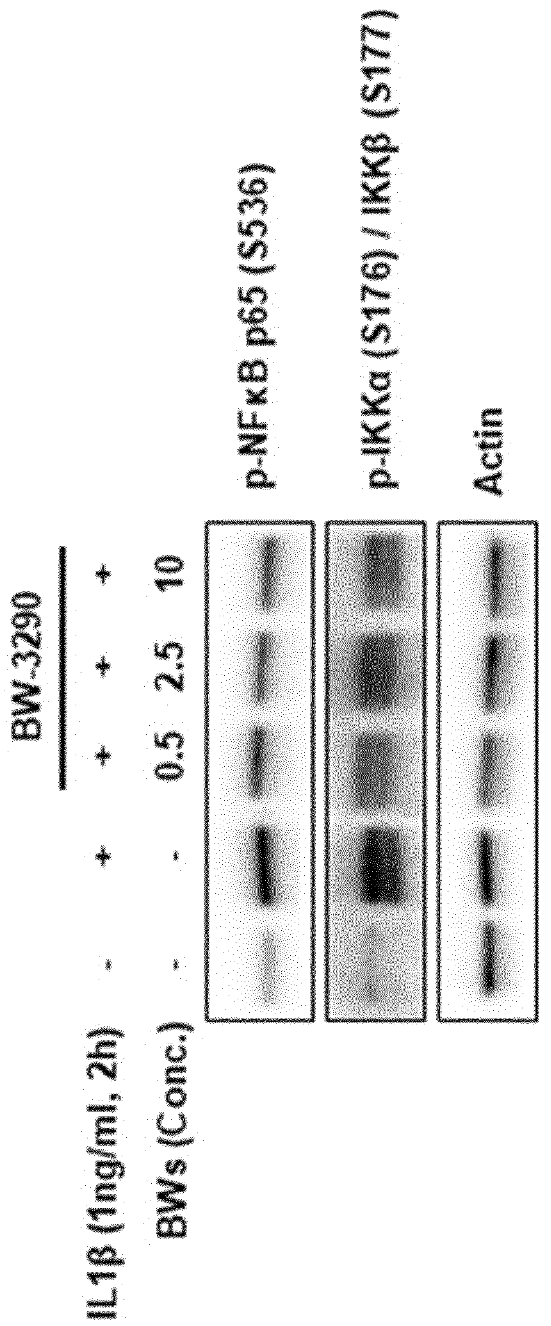

IL1β (1ng/ml, 2h)
BWs (Conc.)
BW-3290
- + + + +
- - 0.5 2.5 10
p-NFκB p65 (S536)
p-IKKα (S176) / IKKβ (S177)
Actin

[FIG. 12]

FIG. 12

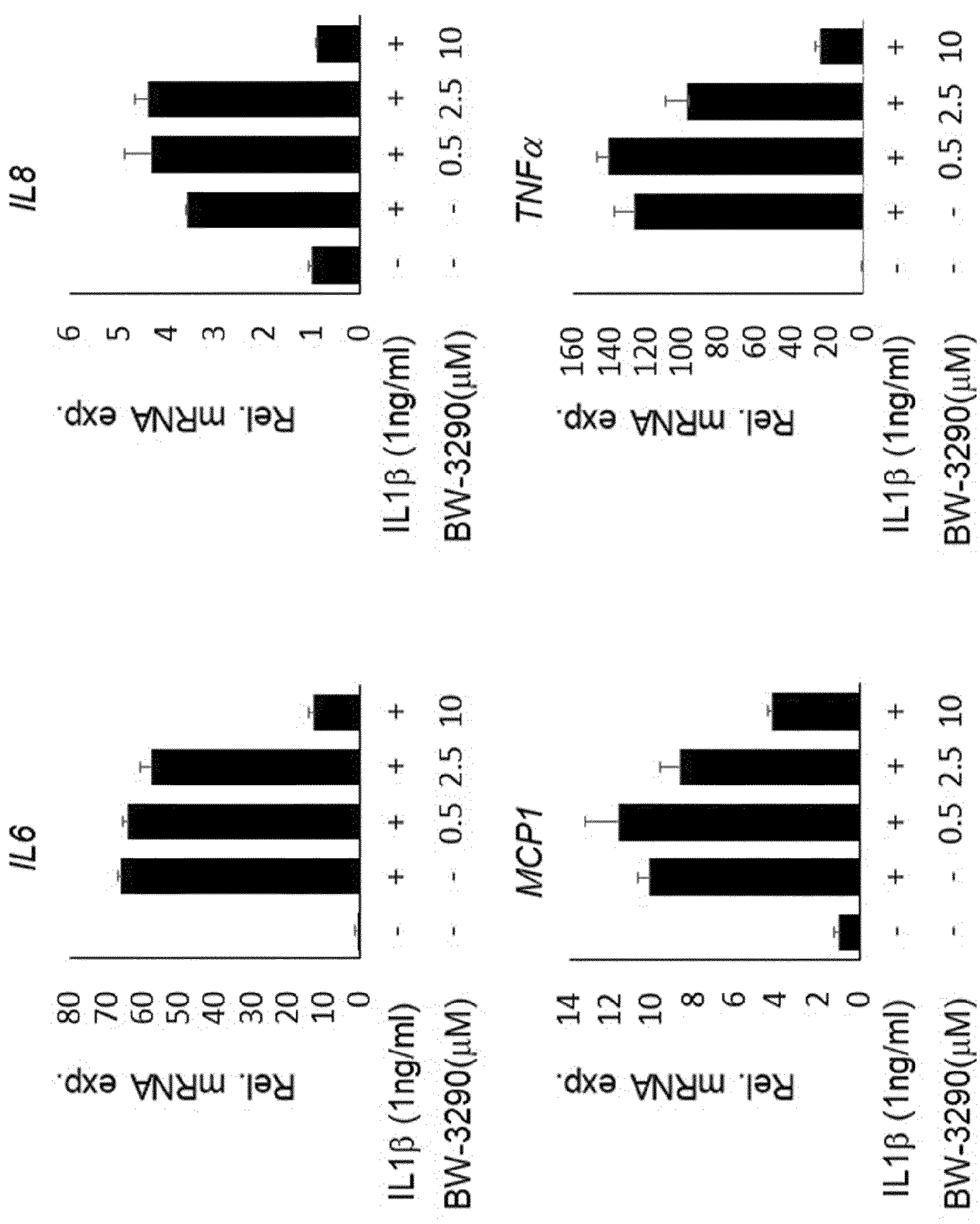

[FIG. 13]

FIG. 13

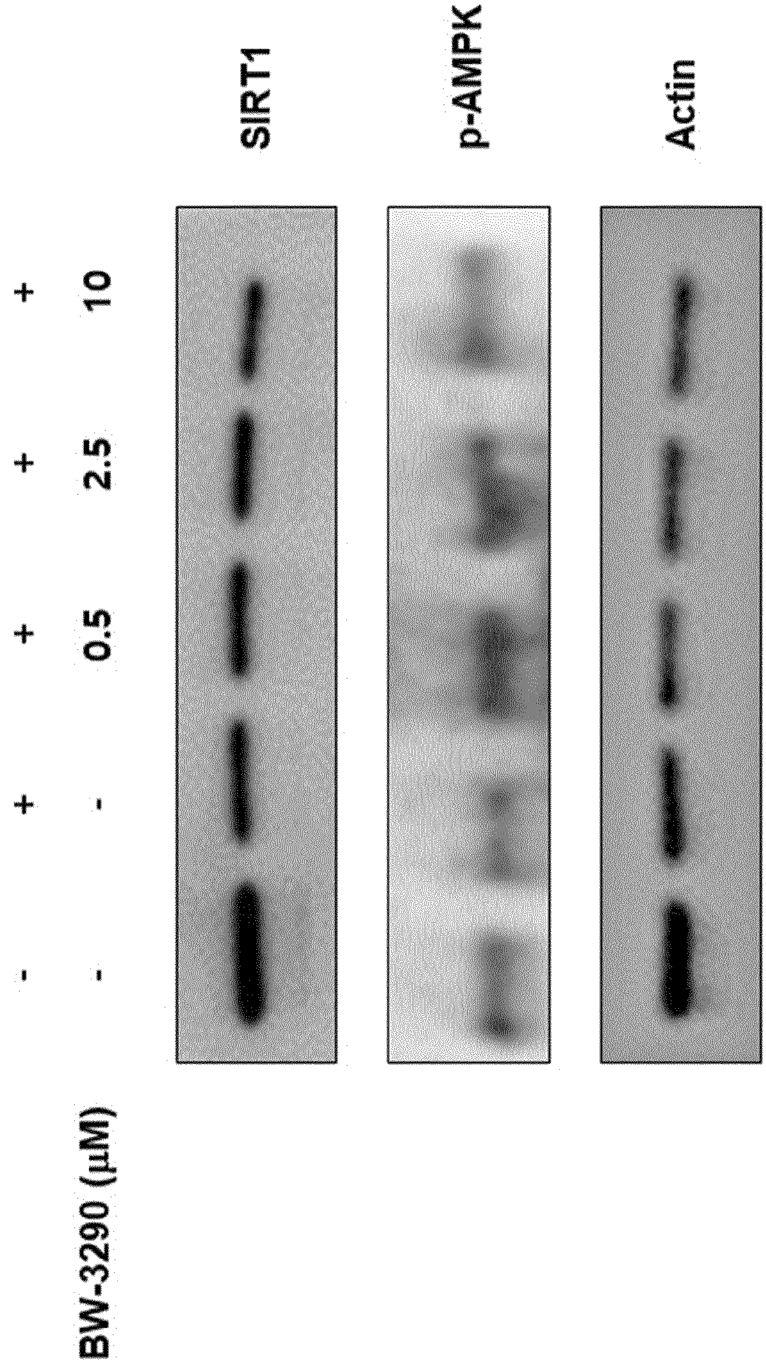

[FIG. 14]

FIG. 14

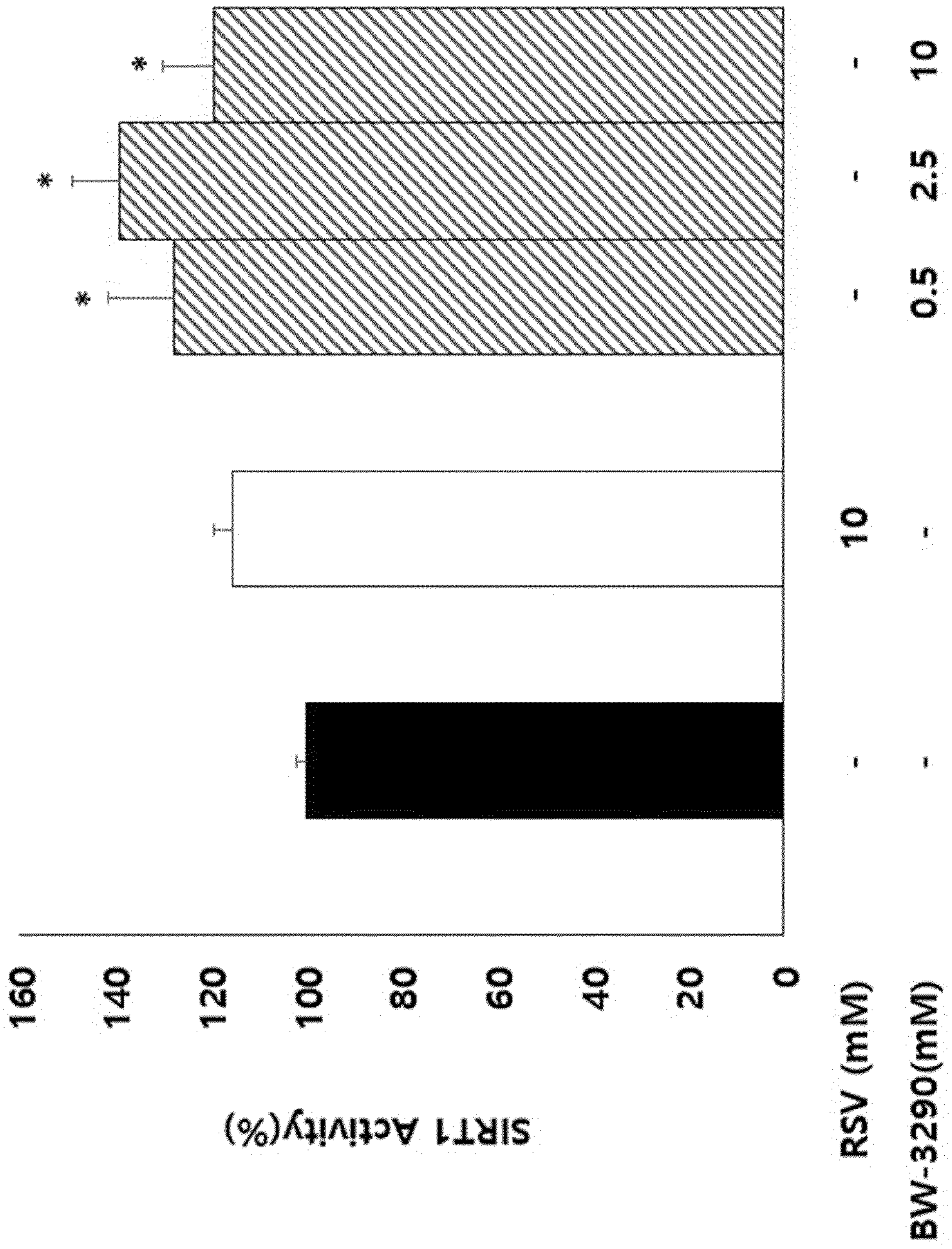
SIRT1 Activity(%)
160
140
120
100
80
60
40
20
0
*
*
*
RSV (mM)
-
10
-
-
-
BW-3290(mM)
-
-
0.5
2.5
10

[FIG. 15]

FIG. 15

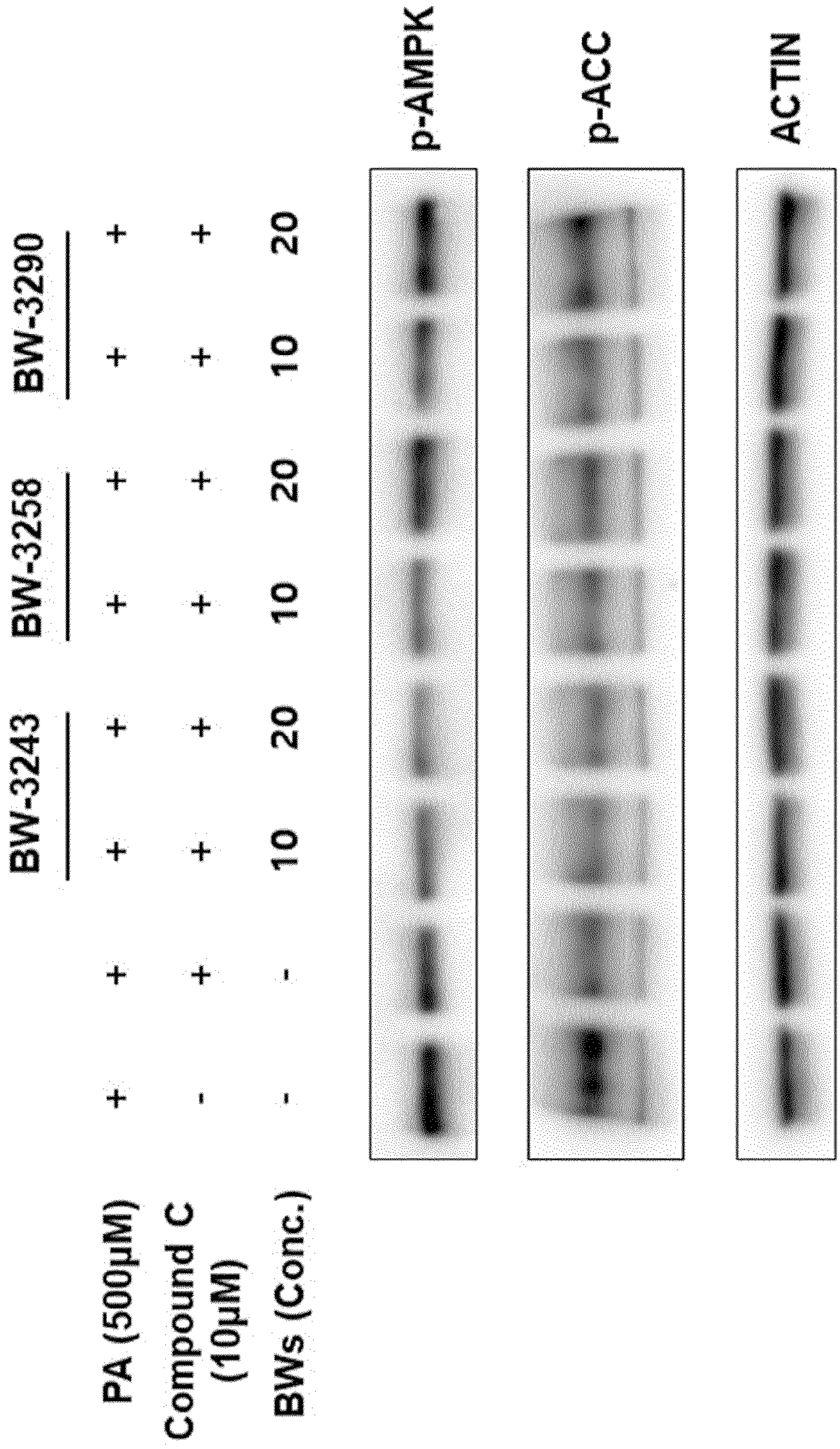
BW-3243
BW-3258
BW-3290
PA (500μM)
Compound C (10μM)
BWs (Conc.)
10
20
10
20
10
20
p-AMPK
p-ACC
ACTIN

[FIG. 16]

FIG. 16

(a)

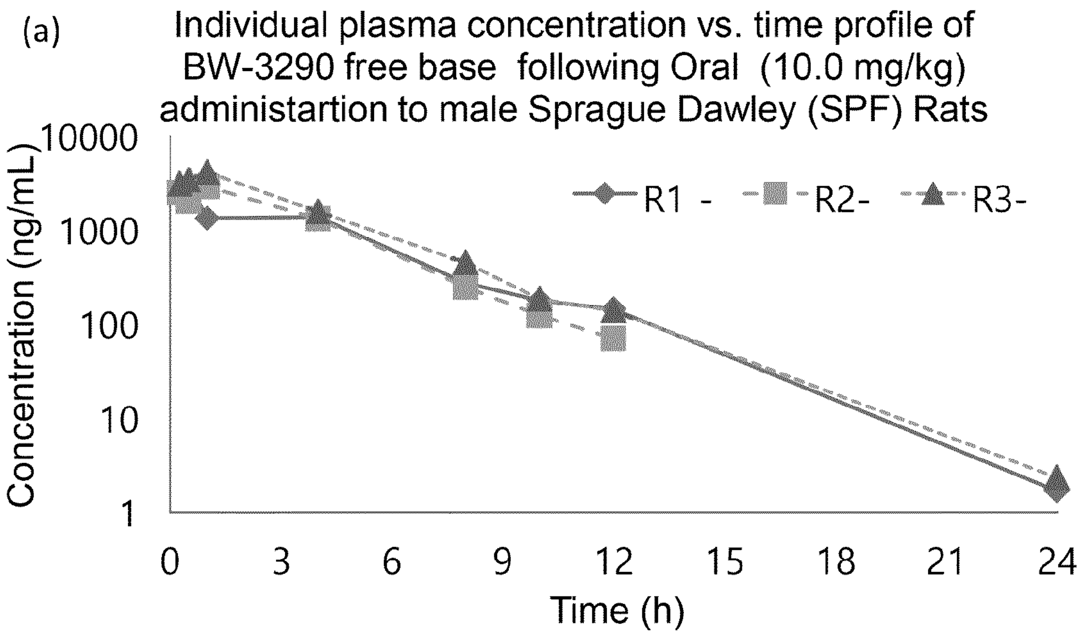


(b)

| Parameter | R1 | R2 | R3 | Mean |
|---|---|---|---|---|
| Dose (mg/kg) | 10.00 | 10.00 | 10.00 | **10.00** |
| $C_{max}$ (ng/mL) | 3019.68 | 2860.49 | 4073.27 | **3317.82** |
| $T_{max}$ (h) | 0.50 | 1.00 | 1.00 | **0.83** |
| $T_{1/2}$ (h) | 2.10 | 2.21 | 2.14 | **2.15** |
| $AUC_{0\text{-}last}$ (ng·h/mL) | 9869.93 | 11086.24 | 15707.74 | **12221.31** |
| $AUC_{0\text{-}inf}$ (ng·h/mL) | 9875.00 | 11308.09 | 15714.91 | **12299.33** |
| $AUC_{Extra}$(%) | 0.05 | 1.96 | 0.05 | **0.69** |
| $MRT_{0\text{-}last}$ (h) | 3.94 | 3.06 | 3.43 | **3.48** |
| Rsq | 0.9900 | 0.9962 | 0.9971 | **0.9944** |
| Bioavailability (%F) | 54.36 | 62.25 | 86.51 | **67.71** |

[FIG. 17]

FIG. 17

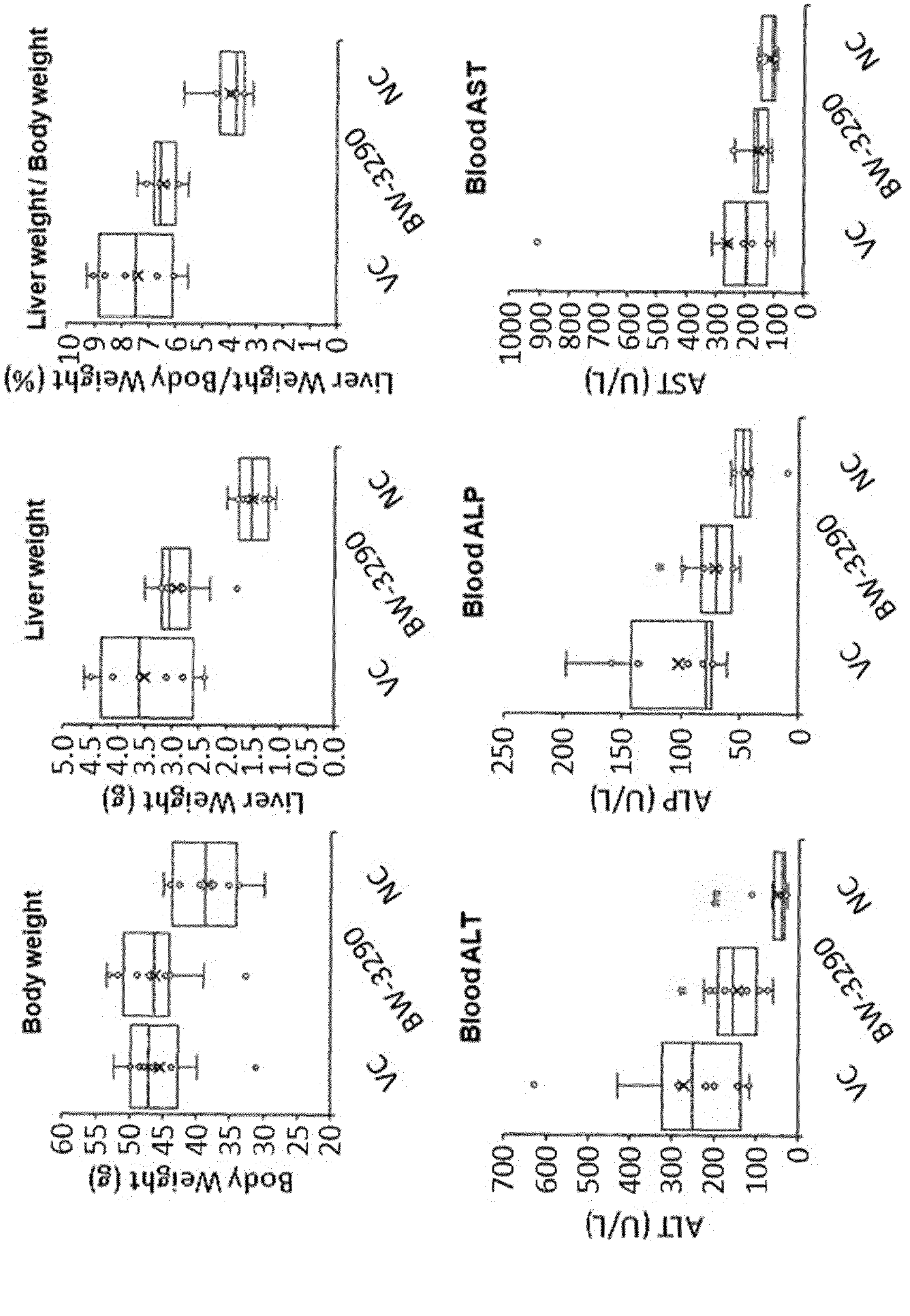
Body weight
Body Weight (g)
Liver weight
Liver Weight (g)
Liver weight / Body weight
Liver Weight/Body Weight (%)
Blood ALT
ALT (U/L)
Blood ALP
ALP (U/L)
Blood AST
AST (U/L)
VC
BW-3290
NC

**[FIG. 18]**

FIG. 18

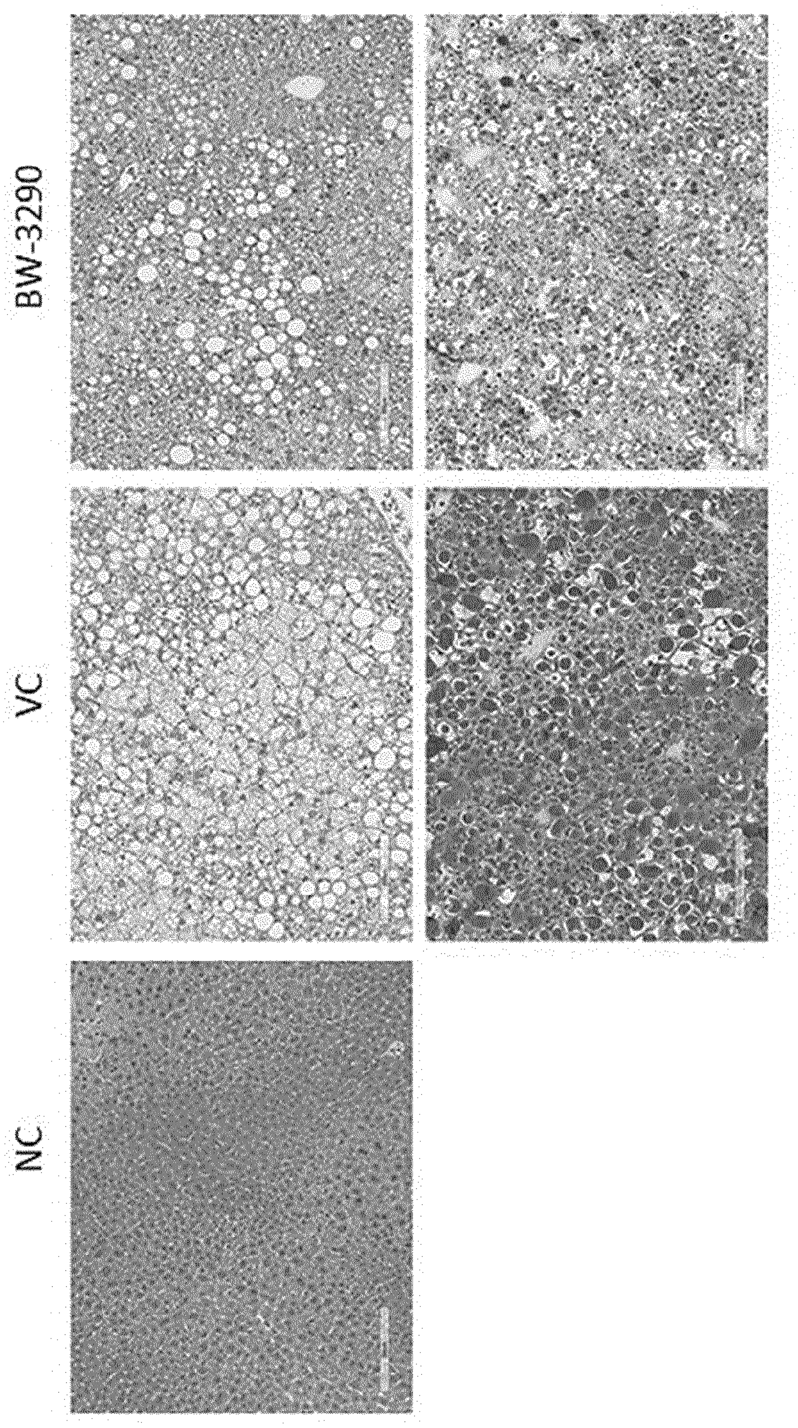

**[FIG. 19]**

FIG. 19

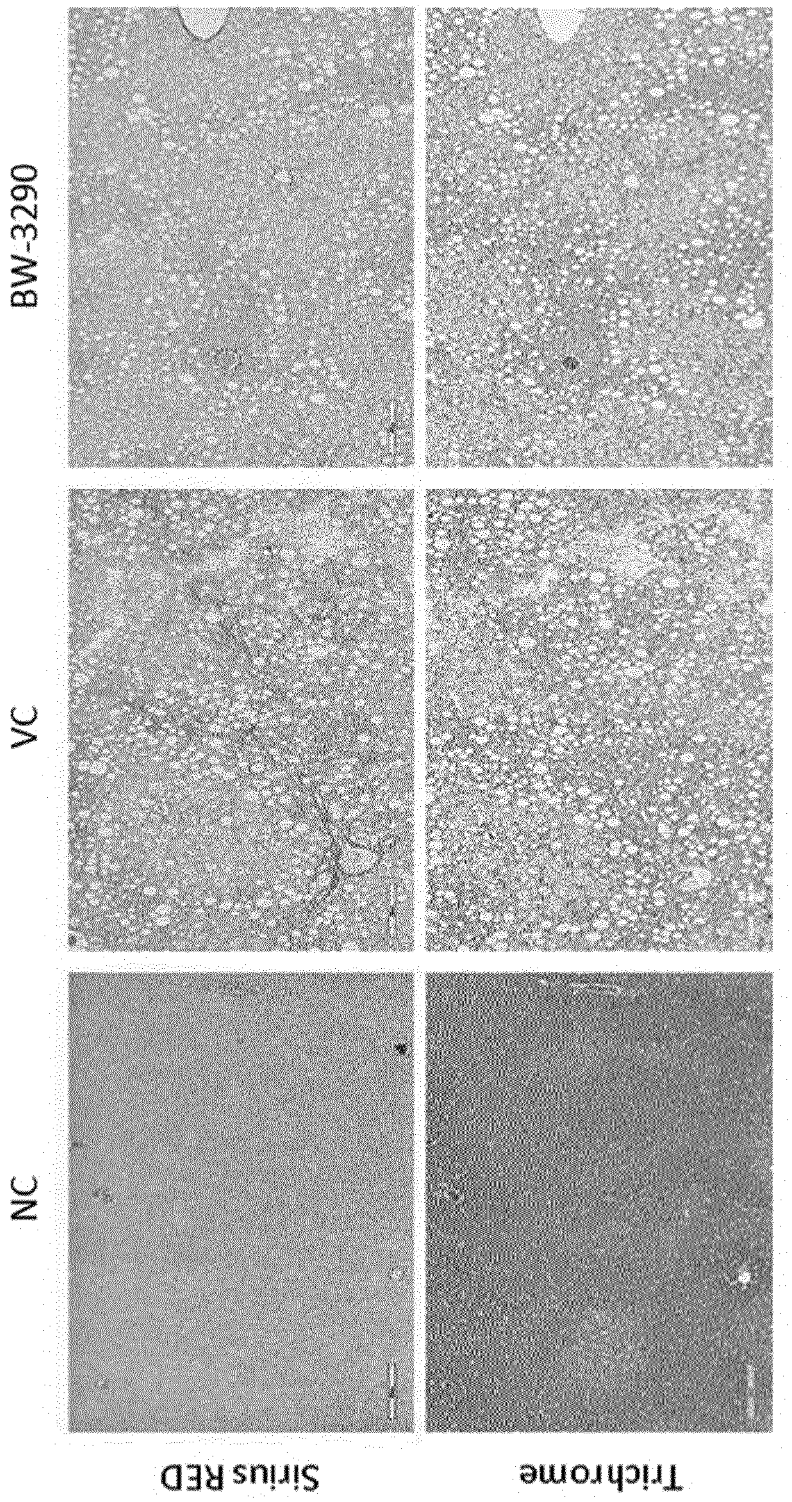

[FIG. 20]

FIG. 20

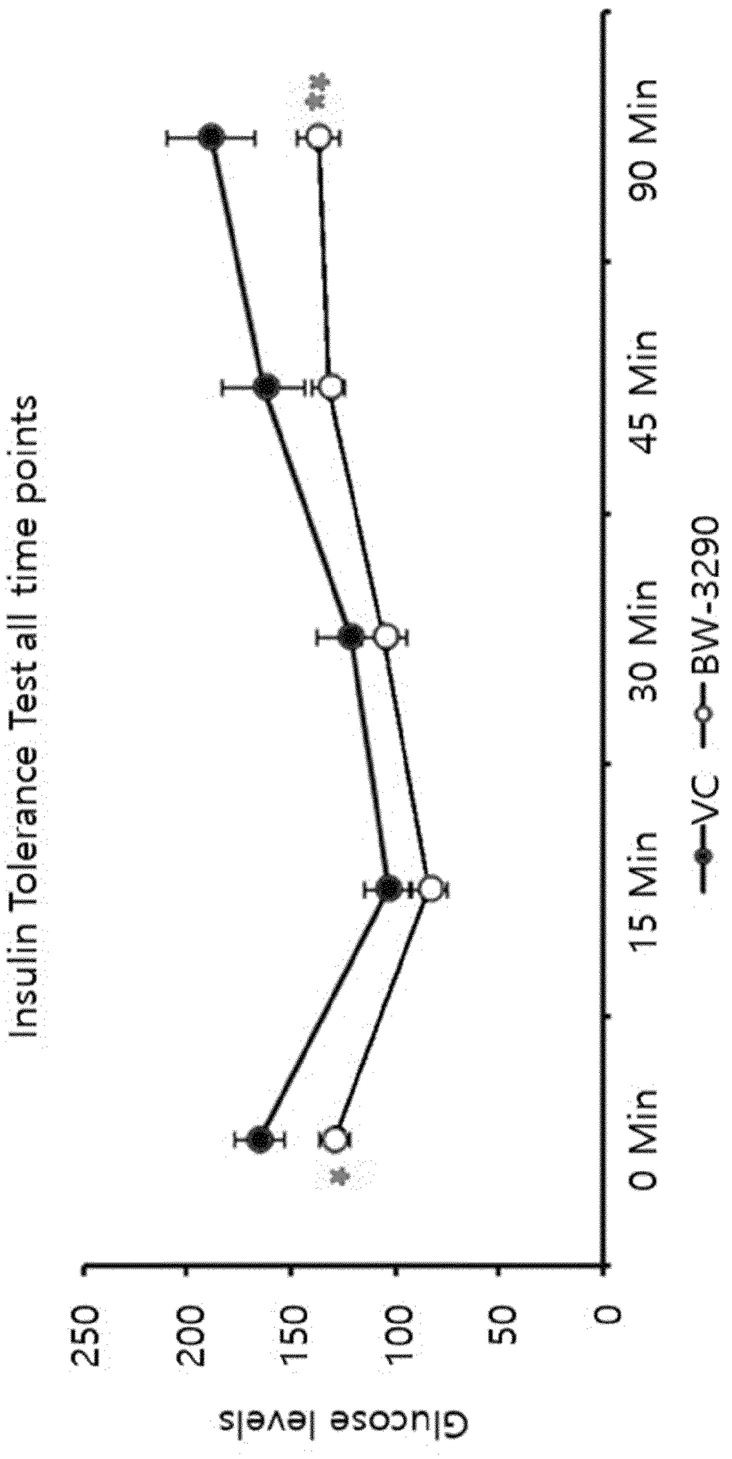
Insulin Tolerance Test all time points
Glucose levels
250
200
150
100
50
0
0 Min
15 Min
30 Min
45 Min
90 Min
*
**
VC
BW-3290

**[FIG. 21]**

FIG. 21

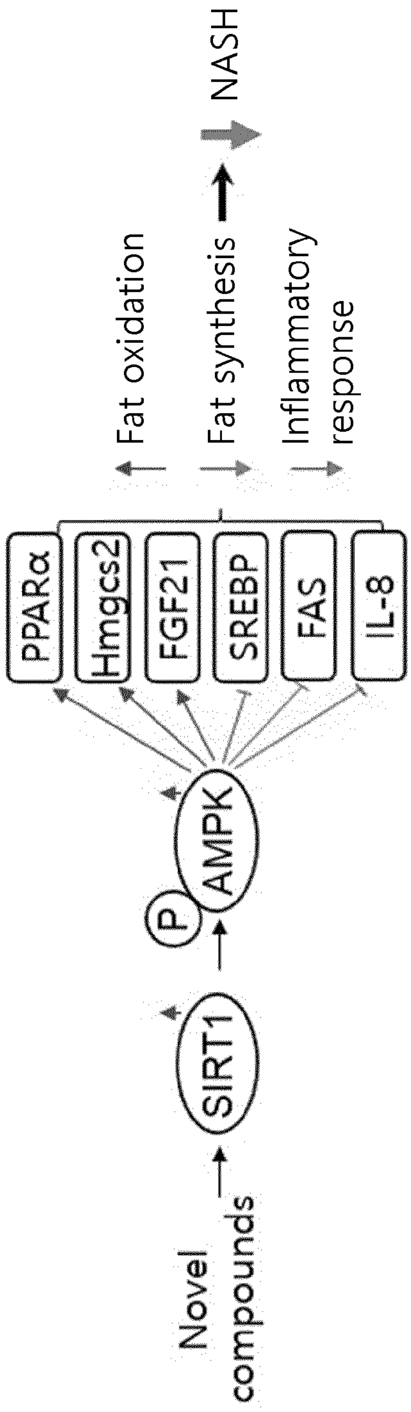
Novel compounds
SIRT1
P
AMPK
PPARα
Hmgcs2
FGF21
SREBP
FAS
IL-8
Fat oxidation
Fat synthesis
Inflammatory response
NASH

[FIG. 22]

FIG. 22

(a)

| Parameter | VC | BW 3290 | NC |
|---|---|---|---|
| Average | 1.5 | 1.2 | 0.0 |
| SD | 0.82 | 0.80 | 0.00 |
| Min | 0.0 | 0.0 | 0.0 |
| Max | 2.0 | 2.0 | 0.0 |
| % Difference | Reference | -16.7% | -100.0% |
| p-value | Reference | 0.4950 | 0.0003 |

(b)

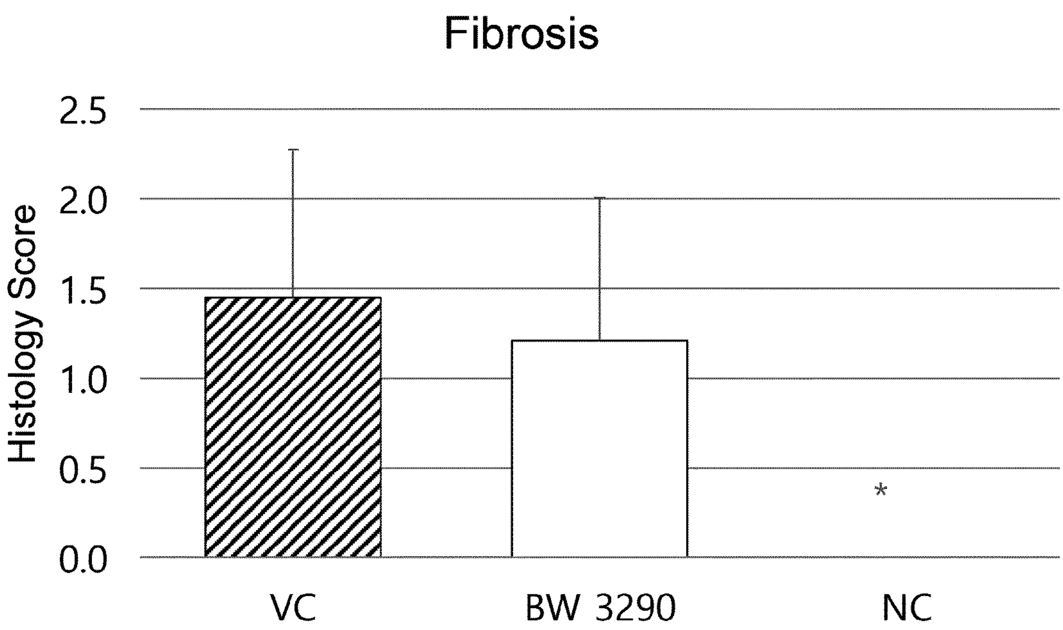

**INTERNATIONAL SEARCH REPORT**

International application No. **PCT/KR2021/000930**

**A. CLASSIFICATION OF SUBJECT MATTER**

**C07D 487/04**(2006.01)i; **A61K 31/52**(2006.01)i; **A61P 1/16**(2006.01)i

According to International Patent Classification (IPC) or to both national classification and IPC

**B. FIELDS SEARCHED**

Minimum documentation searched (classification system followed by classification symbols)

C07D 487/04(2006.01); A61K 31/519(2006.01); A61K 31/52(2006.01); A61P 1/16(2006.01); C07D 495/04(2006.01); C12N 9/16(2006.01); C12Q 1/44(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 퀴나졸리논 유도체(quinazolinone derivative), S형(S form), R형(R form), SIRT1 활성제(Sirtuin 1 activator), AMPK 활성제(AMP-activated protein kinase), 지질 대사질환(lipid metabolism disease), 비알콜성 지방간(non-alcoholic fatty liver disease, NAFLD), 비알콜성 지방간염(non-alcoholic steatohepatitis, NASH), 간섬유화(liver fibrosis)

**C. DOCUMENTS CONSIDERED TO BE RELEVANT**

| Category* | Citation of document, with indication, where appropriate, of the relevant passages | Relevant to claim No. |
|---|---|---|
| X | WO 2011-011550 A1 (CALISTOGA PHARMACEUTICALS INC.) 27 January 2011 (2011-01-27)<br>See claims 1 and 3-5; page 25; and paragraph [0076]. | 1-6 |
| A | WO 2019-036430 A1 (CAMP4 THERAPEUTICS CORPORATION) 21 February 2019 (2019-02-21)<br>See claims 1, 9, 28 and 29. | 1-6 |
| A | US 2006-0079538 A1 (HALLAHAN, D. et al.) 13 April 2006 (2006-04-13)<br>See paragraphs [0083], [0115] and [0123]. | 1-6 |
| A | WO 2008-064018 A1 (ELI LILLY & CO.) 29 May 2008 (2008-05-29)<br>See abstract; and page 29, compound 4b. | 1-6 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 May 2021** | **26 May 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No. **PCT/KR2021/000930**

**C. DOCUMENTS CONSIDERED TO BE RELEVANT**

| Category* | Citation of document, with indication, where appropriate, of the relevant passages | Relevant to claim No. |
|---|---|---|
| PX | CN 111840297 A (TIANJIN JIKUN PHARMACEUTICAL TECH. CO., LTD.) 30 October 2020 (2020-10-30) See claims 1-7. | 1-6 |

Form PCT/ISA/210 (second sheet) (July 2019)

**INTERNATIONAL SEARCH REPORT**
**Information on patent family members**

International application No.
**PCT/KR2021/000930**

| Patent document cited in search report | Publication date (day/month/year) | Patent family member(s) | Publication date (day/month/year) |
|---|---|---|---|
| WO 2011-011550 A1 | 27 January 2011 | AU 2010-276160 A1 | 09 February 2012 |
| | | BR 112012001325 A2 | 02 May 2017 |
| | | CA 2768843 A1 | 27 January 2011 |
| | | CN 102647987 A | 22 August 2012 |
| | | EA 201270184 A1 | 30 August 2012 |
| | | EP 2456443 A1 | 30 May 2012 |
| | | JP 2013-500257 A | 07 January 2013 |
| | | KR 10-2012-0049281 A | 16 May 2012 |
| | | MX 2012000817 A | 08 May 2012 |
| | | US 2012-0178767 A1 | 12 July 2012 |
| | | US 8691829 B2 | 08 April 2014 |
| WO 2019-036430 A1 | 21 February 2019 | CA 3072346 A1 | 21 February 2019 |
| | | CA 3095040 A1 | 10 October 2019 |
| | | CN 111094581 A | 01 May 2020 |
| | | CN 111971063 A | 20 November 2020 |
| | | EP 3668993 A1 | 24 June 2020 |
| | | EP 3668993 A4 | 12 May 2021 |
| | | EP 3773618 A1 | 17 February 2021 |
| | | JP 2020-530858 A | 29 October 2020 |
| | | US 2020-0208128 A1 | 02 July 2020 |
| | | WO 2019-195855 A1 | 10 October 2019 |
| | | WO 2020-037069 A1 | 20 February 2020 |
| | | WO 2020-081329 A1 | 23 April 2020 |
| US 2006-0079538 A1 | 13 April 2006 | CA 2566436 A1 | 01 December 2005 |
| | | CA 2566436 C | 10 May 2011 |
| | | CA 2730540 A1 | 01 December 2005 |
| | | EP 1750714 A1 | 14 February 2007 |
| | | WO 2005-112935 A1 | 01 December 2005 |
| WO 2008-064018 A1 | 29 May 2008 | AU 2007-323836 A1 | 29 May 2008 |
| | | AU 2007-323836 B2 | 18 April 2013 |
| | | CA 2669399 A1 | 29 May 2008 |
| | | CN 101605797 A | 16 December 2009 |
| | | EP 2097422 A1 | 09 September 2009 |
| | | EP 2097422 A4 | 14 April 2010 |
| | | EP 2441768 A1 | 18 April 2012 |
| | | JP 2010-509373 A | 25 March 2010 |
| | | JP 5313909 B2 | 09 October 2013 |
| | | KR 10-2009-0087027 A | 14 August 2009 |
| | | NZ 576997 A | 24 February 2012 |
| | | SG 178786 A1 | 29 March 2012 |
| | | US 2010-0216820 A1 | 26 August 2010 |
| | | WO 2008-064018 A8 | 07 August 2008 |
| CN 111840297 A | 30 October 2020 | None | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 101932146

### Non-patent literature cited in the description


- **SINGLETON et al.** Dictionary of Microbiology and Molecular Biology. 1994
- The Cambridge Dictionary of Science and Technology. 1988
- The Glossary of Genetics. Springer Verlag, 1991
- **HALE ; MARHAM.** The Harper Collins Dictionary of Biology. 1991
- **DING RB ; BAO J ; DENG CX.** *Int J Biol Sci.,* 2017, vol. 13 (7), 852-867
- **PURUSHOTHAM A et al.** *Cell Metab.,* 2009, vol. 9 (4), 327-38
- **CHOI SE ; KWON S ; SEOK S et al.** *Mol Cell Biol.,* 2017, vol. 37 (15), e00006-17
- **WING RR ; GOLDSTEIN MG ; ACTON KJ et al.** Behavioral science research in diabetes: lifestyle changes related to obesity, eating behavior, and physical activity. *Diabetes Care,* 2001, vol. 24 (1), 117-123
- **JEON SM.** Regulation and function of AMPK in physiology and diseases. *Exp Mol Med.,* 15 July 2016, vol. 48 (7), e245
- **WINDER WW ; HARDIE DG.** AMP-activated protein kinase, a metabolic master switch: possible roles in type 2 diabetes. *Am J Physiol.,* 1999, vol. 277 (1), E1-E10
- **BATALLER R ; BRENNER DA.** Liver fibrosis. *J Clin Invest.,* April 2005, vol. 115 (4), 1100
- *J Clin Invest.,* 2005, vol. 115 (2), 209-218